# EUROPEAN PATENT APPLICATION

(11) **EP 4 772 238 A1**
(43) Date of publication of application: **08.07.2026**
(21) Application number: 24881767.8
(22) Date of filing: 25.10.2024
(51) Int. Cl.: A61N 5/10

(54) **RADIOTHERAPY SYSTEM, AND DOSE DELIVERY METHODS AND CONTROL METHODS THEREOF**

(30) Priority: 26.10.2023 CN 202322892767 U; 26.10.2023 CN 202311401922; 26.10.2023 CN 202311403475; 26.10.2023 CN 202311404154
(71) Applicant: Our United Corporation, Xi'an City, Shaanxi 710018 (CN)
(72) Inventor: GUO, Zhao, Xi'an City, Shaanxi 710018 (CN)
(74) Representative: Cabinet Beau de Loménie
(86) International application number: PCT/CN2024/127483
(87) International publication number: WO 2025/087408

(57) **Abstract**

The present application relates to the technical field of radiotherapy. Provided are a radiotherapy system, and dose delivery methods and control methods thereof. The radiotherapy system comprises: a radiation source, a treatment bed and a frame, the radiation source being used for emitting radiation beams, and the radiation source being mounted on the frame, and the treatment bed being used for carrying a patient and moving to a preset position. The radiotherapy system further comprises a first frame driving device and a second frame driving device, the first frame driving device being used for driving the frame to rotate around a frame rotation axis, the second frame driving device being used for driving the frame to rotate with a first axis as an rotation axis, the first axis being perpendicular to the horizontal plane, and the second frame driving device being higher than the lowest point of rotation of the frame. Therefore, a patient can obtain a space for therapy that is fixed relative to the floor, and does not feel high-speed rotation of the frame, thereby enabling the patient to have good experience. Non-coplanar irradiation is achieved by swinging the frame, and during a therapy process for a patient, the frame swings to adjust the non-coplanar incident angle and the patient does not move any more after patient positioning, thereby achieving high precision for positioning target sites of the patient and enabling the patient to have good experience during therapy.

## Description

This application claims priority to Chinese Patent Application No. 202322892767.7, 202311403475.0, 202311404154.2, and 202311401922.9, filed on October 26, 2023, and entitled "RADIOTHERAPY SYSTEM," "COMPOSITE COLLIMATOR AND CONTROL METHOD THEREOF, RADIOTHERAPY SYSTEM AND DOSE DELIVERY METHOD THEREOF," "RADIOTHERAPY SYSTEM AND DOSE DELIVERY METHOD THEREOF," and "COMPOSITE COLLIMATOR AND CONTROL METHOD THEREOF, RADIOTHERAPY SYSTEM AND DOSE DELIVERY METHOD THEREOF," the entire contents of which are incorporated herein by reference.

### TECHNICAL FIELD

This application relates to the field of radiotherapy technology, specifically to a radiotherapy system, a dose delivery method therefor, and a control method thereof.

### BACKGROUND

Radiotherapy can locally treat tumors with radiation, thereby eliminating and curing the primary or metastatic foci of local tumors. Conventional radiotherapy techniques are categorized into coplanar irradiation and non-coplanar irradiation; wherein the coplanar irradiation refers to the beam axes of all radiation fields being in the same plane, while the non-coplanar irradiation involves one or more radiation fields having beam axes that are not in the plane formed by the beam axes of other radiation fields. The non-coplanar irradiation can achieve a more satisfactory dose distribution and is therefore widely used in actual treatment.

Existing non-coplanar techniques are mainly achieved by the rotation of the treatment couch. In this case, after the patient is positioned on the treatment couch, the treatment couch needs to be moved, which poses a risk of target volume variation. Furthermore, frequent movement of the treatment couch during treatment to change non-coplanar angles results in a poor treatment experience for the patient.

### SUMMARY

An object of embodiments of the present disclosure is to provide a radiotherapy system, a dose delivery method therefor, and a control method thereof, which can achieve non-coplanar irradiation without moving the treatment couch, thereby improving the patient's treatment experience.

In one aspect of the embodiments of the present disclosure, a radiotherapy system is provided. The radiotherapy system includes: a radiation source, a treatment couch, and a gantry, wherein the radiation source is configured to emit radiation beams and is mounted on the gantry; and the treatment couch is configured to carry a patient and move the patient to a predetermined position. The radiotherapy system further includes a first gantry driving device and a second gantry driving device, wherein the first gantry driving device is configured to drive the gantry to rotate about a gantry rotation axis, and the second gantry driving device is configured to drive the gantry to rotate about a first axis as a rotation axis, the first axis being perpendicular to the horizontal plane, and the second gantry driving device is higher than a lowest point of the rotation of the gantry.

In another aspect of the embodiments of the present disclosure, a dose delivery method for a radiotherapy system is provided, applicable for dose delivery for the aforementioned radiotherapy system. The method includes: positioning a patient within a treatment space of the gantry by driving the treatment couch; making the treatment couch and the gantry form different angles by driving the gantry to rotate about the first axis as the rotation axis, so as to allow radiation beams to be emitted from different angles, the first axis being perpendicular to a horizontal plane; and making the radiation beams be directed to a target volume from different orientations by driving the gantry to rotate about the gantry rotation axis.

In another aspect of the embodiments of the present disclosure, a dose delivery method for a radiotherapy system is provided, applicable for dose delivery for the radiotherapy system. The method includes: acquiring treatment plan information for a target subject; and making radiation beams guided to a target volume pass through the first collimating channel of the block and the collimator channel, or making the radiation beams only pass through the collimator channel by controlling the compound collimator of a treatment head based on the treatment plan.

In another aspect of the embodiments of the present disclosure, a dose delivery method for a radiotherapy system is provided, applicable for dose delivery for the radiotherapy system. The method includes: acquiring treatment plan information for a target subject; and making radiation beams guided to a target volume pass through the second collimating channel of the second collimating body and a collimator channel formed by the first leaf group, or making the radiation beams pass through the collimator channel formed by the first leaf group and/or a collimator channel formed by the second leaf group by controlling the compound collimator of a treatment head based on the treatment plan information.

In another aspect of the embodiments of the present disclosure, a control method for a radiotherapy system is provided, applicable for controlling the radiotherapy system. The method includes: acquiring irradiation information for a target object; in response to determining that a focused radiation field is adopted for irradiation of the target object based on the irradiation information, controlling the block to move to enable the beam center axis to pass through the first collimating channel and collimator channel, so as to make the radiation beams emitted by the radiation source form the focused radiation field in a target volume in response to passing through the first collimating channel and collimator channel; and in response to determining that a conformal radiation field is adopted for irradiation of the target object based on the irradiation information, controlling the block to move to a evasive position, so as to make the radiation beams emitted by the radiation source form the conformal radiation field in response to passing through the collimator channel.

In another aspect of the embodiments of the present disclosure, a control method for a radiotherapy system is provided, applicable for controlling the radiotherapy system. The method includes: acquiring irradiation information for a target object; in response to determining that a focused radiation field is adopted for irradiation of the target object based on the irradiation information, controlling the second collimating body and the first leaf group to move to enable the beam center axis to pass through the second collimating channel of the second collimating body and the collimator channel of the first leaf group, so as to make the radiation beams emitted by the radiation source form the focused radiation field in a target volume in response to passing through the second collimating channel and the collimator channel; and in response to determining that a conformal radiation field is adopted for irradiation of the target object based on the irradiation information, controlling the second collimating body to move to a evasive position, so as to make the radiation beams emitted by the radiation source form the conformal radiation field in response to passing through a collimator channel formed by the first leaf group and a collimator channel formed by the second leaf group.

### BRIEF DESCRIPTION OF DRAWINGS

For clearer illustration of the technical solutions of the embodiments of the present disclosure, the accompanying drawings required for use in the embodiments of the present disclosure are briefly introduced below. It should be understood that the following drawings only illustrate some embodiments of the present disclosure and should not be regarded as limiting the scope. For those skilled in the art, other relevant drawings can be obtained based on these drawings without creative effort.
FIG. 1 is a simplified schematic diagram of a radiotherapy system according to some embodiments of the present disclosure;
FIG. 2 is a simplified schematic diagram of a radiotherapy system according to some embodiments of the present disclosure;
FIG. 3 is a simplified schematic diagram of a radiotherapy system according to some embodiments of the present disclosure;
FIG. 4 is a schematic diagram of a compound collimator in an open position according to some embodiments of the present disclosure;
FIG. 5 is a schematic diagram of a compound collimator in a closed position according to some embodiments of the present disclosure;
FIG. 6 is a schematic diagram of a collimator structure of a compound collimator according to some embodiments of the present disclosure;
FIG. 7 is a diagram showing the correspondence between a block channel and a first collimating channel of a compound collimator according to some embodiments of the present disclosure;
FIG. 8 is a diagram showing the correspondence between a block channel and a first collimating channel of a compound collimator according to some embodiments of the present disclosure;
FIG. 9 is a diagram showing the correspondence between a block channel and a first collimating channel of a compound collimator according to some embodiments of the present disclosure;
FIG. 10 is a diagram showing the correspondence between a block channel and a first collimating channel of a compound collimator according to some embodiments of the present disclosure;
FIG. 11 is a diagram showing the correspondence between a block channel and a first collimating channel of a compound collimator according to some embodiments of the present disclosure;
FIG. 12 is a rotational sectional view of FIG. 4;
FIG. 13 is another embodiment diagram of FIG. 12;
FIG. 14 is a schematic diagram of the field formation principle of the compound collimator according to some embodiments of the present disclosure;
FIG. 15 is a schematic diagram of the field formation principle of the compound collimator according to some embodiments of the present disclosure;
FIG. 16 is a schematic diagram of the compound collimator in a closed position according to some embodiments of the present disclosure;
FIG. 17 is a schematic diagram of the compound collimator in an open position according to some embodiments of the present disclosure;
FIG. 18 is a schematic diagram of the compound collimator in a closed position according to some embodiments of the present disclosure;
FIG. 19 is a rotational sectional view of FIG. 17;
FIG. 20 is another embodiment diagram of FIG. 19;
FIG. 21 is a schematic diagram of the swinging of the treatment couch in the radiotherapy system according to some embodiments of the present disclosure;
FIG. 22 is a schematic diagram of the local structure of the radiotherapy system according to some embodiments of the present disclosure;
FIG. 23 is a schematic diagram of the local structure of the radiotherapy system according to some embodiments of the present disclosure;
FIG. 24 is a schematic diagram of the local structure of the radiotherapy system according to some embodiments of the present disclosure;
FIG. 25 is a schematic diagram of the structure of the radiotherapy system according to some embodiments of the present disclosure;
FIG. 26 is a schematic diagram of the gantry swing of the radiotherapy system according to some embodiments of the present disclosure;
FIG. 27 is a schematic diagram of the local structure of the radiotherapy system according to some embodiments of the present disclosure;
FIG. 28 is a schematic diagram of the local structure of the radiotherapy system according to some embodiments of the present disclosure;
FIG. 29 is a schematic diagram of the local structure of the radiotherapy system according to some embodiments of the present disclosure;
FIG. 30 is a schematic diagram of the local structure of the radiotherapy system according to some embodiments of the present disclosure;
FIG. 31 is a schematic diagram of the local structure of the radiotherapy system according to some embodiments of the present disclosure;
FIG. 32 is a schematic diagram of the local structure of the radiotherapy system according to some embodiments of the present disclosure;
FIG. 33 is a schematic diagram of the local structure of the radiotherapy system according to some embodiments of the present disclosure;
FIG. 34 is a schematic diagram of the local structure of the radiotherapy system according to some embodiments of the present disclosure;
FIG. 35 is a schematic diagram of the local structure of the radiotherapy system according to some embodiments of the present disclosure;
FIG. 36 is a schematic diagram of the local structure of the radiotherapy system according to some embodiments;
FIG. 37 is a flowchart of a control method for a compound collimator according to some embodiments of the present disclosure;
FIG. 38 is a flowchart of a dose delivery method for a radiotherapy system according to some embodiments of the present disclosure;
FIG. 39 is a flowchart of a dose delivery method for a radiotherapy system according to some embodiments;
FIG. 40 is a flowchart of a dose delivery method for a radiotherapy system according to some embodiments of the present disclosure;
FIG. 41 is a flowchart of a control method for a compound collimator according to some embodiments of the present disclosure;
FIG. 42 is a flowchart of a dose delivery method for a radiotherapy system according to some embodiments of the present disclosure; and
FIG. 43 is a flowchart of a dose delivery method for a radiotherapy system according to some embodiments of the present disclosure.

### DETAILED DESCRIPTION

The technical solutions in the embodiments of the present disclosure are clearly and comprehensively described hereafter in conjunction with the accompanying drawings.

In the description of the present disclosure, it should be noted that the terms "internal", "external", and the like indicate the orientation or positional relationship based on the orientation or positional relationship shown in the accompanying drawings, or the orientation or positional relationship that is customarily placed when the product of the present disclosure is used. This is only for the convenience of describing the present disclosure and simplifying the description, and does not indicate or imply that the device or component referred to must have a specific orientation, be constructed and operated in a specific orientation. Therefore, it cannot be understood as a limitation on the present disclosure. In addition, terms such as "first", "second", and the like are only used for distinction in description and cannot be understood as indicating or implying relative importance.

It should also be noted that unless otherwise explicitly specified and limited, the terms "setting" and "connection" should be broadly understood. For example, they can be fixed connections, detachable connections, or integrally connected; they can be directly connected, indirectly connected through an intermediate medium, or connected internally within two components. For those skilled in the art, the specific meanings of the aforementioned terms in the present disclosure can be understood based on specific situations.

Radiotherapy refers to the local treatment of tumors using radiation, aiming to eliminate and cure the primary or metastatic foci of local tumors.

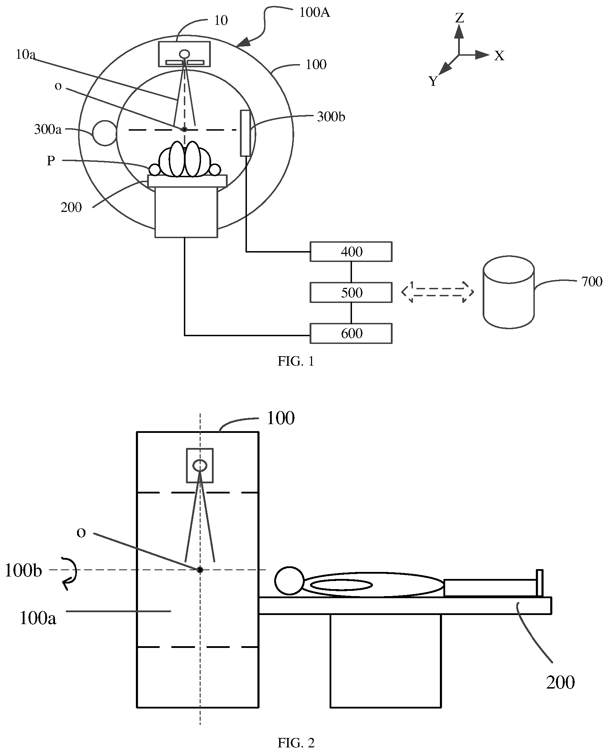

The embodiments of the present disclosure provide a radiotherapy system. As shown in FIGS. 1 to 3, the radiotherapy system includes a gantry 100, a treatment head, and a treatment couch 200; wherein the treatment couch 200 is configured to support and move the patient; the treatment head includes a radiation source 10 and a collimator, the radiation source 10 emits radiation beams 10a towards the target volume, and the collimator shapes the radiation beams 10a, such that the radiation beams 10a guided to the target volume forms a predetermined radiation field of a specific shape, ensuring that the rays irradiating the patient's part are adapted to the tumor; and the gantry 100 is configured to install various radiotherapy components and drives the treatment head to rotate about the gantry rotation axis 100b, so as to allow the radiation beams 10a be emitted from different angles.

In some embodiments, as shown in FIG. 1, the gantry 100, the treatment head, and the treatment couch 200, etc., constitute an integrated radiation delivery device 100A; and the radiotherapy system further includes a master control system 500, a slave control system 400, a treatment planning system 600, and a memory 700. In some embodiments, the radiation delivery device 100A, the master control system 500, the slave control system 400, the treatment planning system 600, and the memory 700 are connected and/or communicate with each other via wireless connections (e.g., network connections), wired connections, or a combination thereof.

In some embodiments, the master control system 500 is capable of generating control instructions for one or more components of the radiotherapy system, such as the slave control system 400, the treatment planning system 600, or the memory 700.

In some embodiments, the slave control system 400 is configured to control the radiation delivery device 100A to perform corresponding actions in response to control instructions generated by the master control system 500.

In some embodiments, the treatment planning system 600 is configured to determine a treatment plan based on the patient's planning images (planning images are images acquired by the imaging device before treatment) and/or based on at least a portion of the object (e.g., tumor) represented in the images acquired by the imaging system.

The memory 700 is configured to store data, instructions, and/or any other information. In some embodiments, the memory 700 stores data obtained from the treatment planning system 600. In some embodiments, the memory 700 stores data and/or instructions used by the master control system 500 to execute the exemplary methods described in the present disclosure.

The radiation source 10 is capable of generating or emitting a radiation beam 10a, and there can be one or more radiation sources 10. In some embodiments, the radiation source is an X-ray radiation source, a gamma-ray radiation source, or other types such as electrons, protons, or heavy ions. The present disclosure does not limit the type of radiation source, and only uses the X-ray radiation source as an example for illustration. In some embodiments, the position of the radiation source 10 relative to the patient and the orientation of the radiation beam 10a relative to the patient are achieved by controlling the movement of the gantry 100 and/or the treatment couch 200.

The treatment couch 200 is configured to support the patient P and is translatable in one or more of three orthogonal directions (shown as X, Y, and Z directions in FIG. 1). In some embodiments, the treatment couch 200 is also rotatable about any one or more of the three axes X, Y, and Z.

The gantry 100 is configured to support the treatment head and is capable of driving the treatment head to rotate about the gantry rotation axis 100b. The gantry rotation axis 100b and the central axis of the radiation beam 10a intersect at the isocenter O.

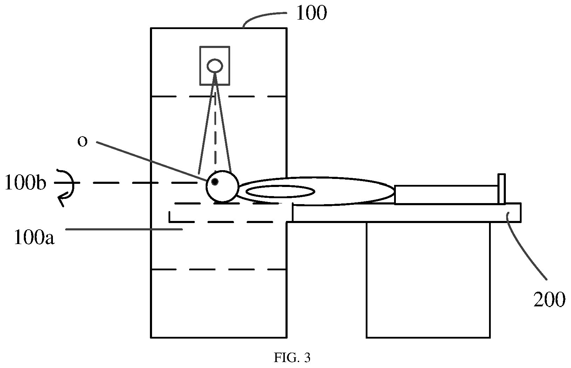

The gantry 100 includes a C-shaped gantry, a roller gantry, etc. In the present disclosure, the roller gantry 100 is taken as an example. As shown in FIGS. 2 and 3, a treatment space 100a is formed in the gantry 100, and the treatment couch 200 enters the treatment space 100a and treats the patient on the treatment couch 200 through various radiotherapy components on the gantry 100.

In some embodiments, the treatment couch 200 and the gantry 100 are relatively deflected, allowing for different angles between the treatment couch 200 and the gantry 100. In this way, the radiation beams 10a are emitted from different angles to achieve coplanar or non-coplanar irradiation, providing a flexible beam arrangement irradiation scheme and enabling patients to have better treatment outcomes. Furthermore, in some embodiments, during the treatment process, the gantry 100 also rotates around the gantry rotation axis 100b, allowing the radiation beams to be directed to the target volume from different orientations with a predetermined radiation field.

In some embodiments, the gantry 100 is also equipped with an imaging system, which is configured to achieve precise radiotherapy. The imaging system includes a tube 300a and a detector 300b. The tube 300a emits imaging beams, which pass through the patient and are received by the detector 300b. By acquiring patient images, the imaging system provides information on the shape, volume, and position of tumors and critical organs. This information is then registered with the images of the treatment plan, allowing for imaging verification of whether the patient has moved or is accurately positioned. In some embodiments, based on the results of the imaging registration verification, adjustments are made to the patient, treatment is stopped, or the treatment plan is adjusted. In some embodiments, some images from the image guidance system are also used to develop treatment plans; and continuous dynamic images are used to observe and evaluate changes in tumor and organ morphology and position caused by physiological movements. In some embodiments, the imaging system is a CT device, a cone-beam CT device, a PET device, a volumetric CT device, an MRI device, or a combination thereof.

In some embodiments, the imaging system includes one tube 300a and one detector 300b, or two tubes 300a and two detectors 300b, namely, a first imaging system and a second imaging system, with the rays of the first and second imaging systems intersecting.

In the case that the system is equipped with a first imaging system and a second imaging system, three-dimensional imaging of the target volume is achieved in one phase of the gantry 100 with the aid of the dual imaging system, enabling real-time monitoring of the lesion location. Simultaneously, the time required for image data acquisition is reduced, enhancing the efficiency of image guidance.

In some embodiments of the present disclosure, the collimator is configured to shape the radiation beams 10a guided to the target volume into a predetermined radiation field of a specific shape. After the generation of rays caused by electrons striking the target, the primary collimator performs preliminary conformal shaping on the radiation beams 10a emitted by the radiation source 10, generally conforming it to a conical or square-conical shape. The present disclosure provides a compound collimator that is capable of constraining the preliminarily shaped radiation beams 10a to a predetermined radiation field within the target volume.

For irradiation with the radiation field, generally, conformal radiation field (Fc) is selected for irradiation on large body tumors, but for small tumors, the precision of focused radiation field (Ff) is higher. In some embodiments, the focused radiation source is further used for local dose enhancement of tumors. To provide a more flexible irradiation plan for clinical applications, the compound collimator provided in the embodiments of the present disclosure is capable of achieving seamless switching between different radiation fields.

In some embodiments, the present disclosure provides a compound collimator for shaping the radiation beams emitted by a radiation source. The compound collimator includes: a collimator and a block that are arranged sequentially along the direction of the beam center axis, wherein the collimator includes a plurality of leaves, and the plurality of leaves forms, by movements, a collimator channel allowing the radiation beam to pass through; and the block is provided with a first collimating channel, and the block is moveable relative to the beam center axis; wherein in the case that the block moves to position the first collimating channel on the beam center axis, the radiation beams form a focused radiation field in the target volume in response to passing through the collimator channel and the first collimating channel; and in the case that the block moves to make the first collimating channel deviates from the beam center axis, the radiation beams form a conformal radiation field in the target volume in response to passing through the collimator channel. By switching between the conformal radiation field and the focused radiation field, a flexible irradiation scheme is provided, thereby improving the dose distribution effect and enhancing the radiation treatment effect.

It should be noted that in this disclosure, "the channel is on the beam center axis" refers to the channel being positioned at a specific location where allows the central axis of the radiation beams to pass through, that is, the beam center axis is able to pass through the channel; "the channel deviates from the beam center axis" in this disclosure refers to the structure containing the channel (such as the aforementioned block) moving to a position where the radiation beams are not blocked from passing through the collimator channel, which is actually an evasive position. That is, in the case that the block moves to the evasive position, the radiation beam is able to pass through the collimator channel. Similar descriptions in the following should be understood in the same way and are not explained individually.

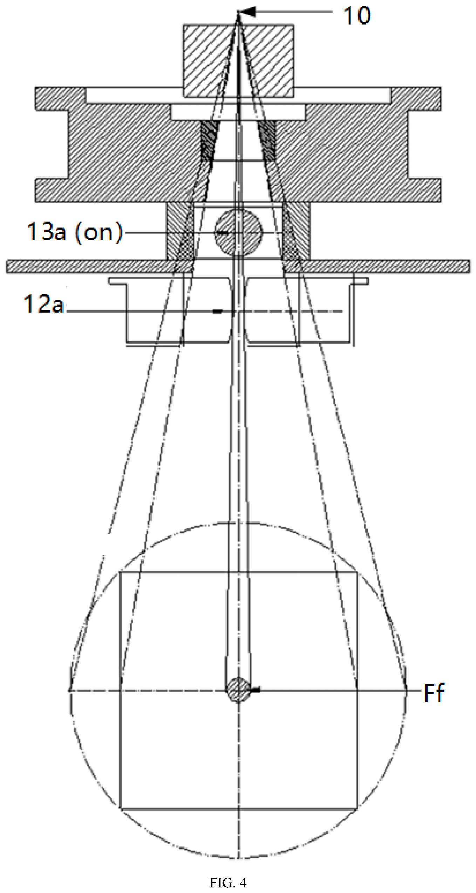

In some embodiments, referring to FIGS. 4 to 7, the embodiments of the present disclosure provide a compound collimator for shaping the radiation beams 10a emitted by the radiation source 10. The compound collimator includes a collimator 12 and a block 13 that are arranged sequentially along the direction of the beam center axis. The radiation beams 10a emitted by the radiation source 10 are shaped into a predetermined radiation field in response to passing through the collimator 12 and the block 13.

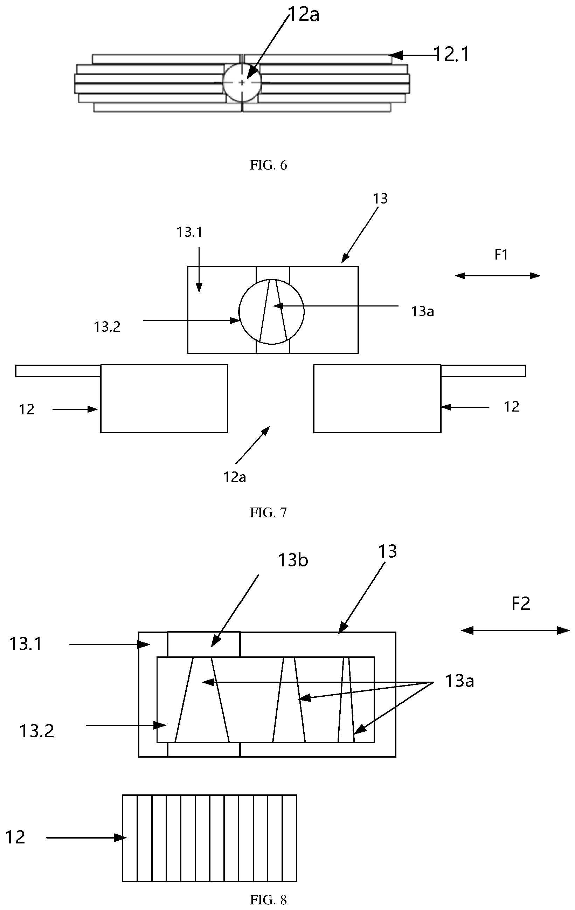

As shown in FIG. 6, the collimator 12 includes a plurality of leaves 12.1, and the leaves 12.1 form, by movements, a collimator channel 12a allowing the radiation beams 10a to pass through. By controlling the movement of the leaves 12.1, a complex shape or an approximately circular radiation field is formed in some embodiments. In some embodiments, the collimator includes leaves, drivers, etc. The specific structure of the collimator is not described in detail in the present disclosure.

In some embodiments, as shown in FIGS. 4 to 7, a first collimating channel 13a is formed in the block 13, and the block 13 is moveable along a first direction F1; wherein in the case that the block 13 moves to position the first collimating channel 13a on the beam center axis, the radiation beams 10a form a focused radiation field Ff in the target volume in response to passing through the collimator channel 12a and the first collimating channel 13a; and in the case that the block 13 moves to make the first collimating channel 13a deviate from the beam center axis, the radiation beams 10a form a conformal radiation field Fc in the target volume in response to passing through the collimator channel 12a. In some embodiments, the second direction F2 is perpendicular to the movement direction of the leaves, and the first direction F1 is parallel to the movement direction of the leaves. In some embodiments, the block also moves along the second direction F2.

In some embodiments, the translation direction of the block body is parallel to the movement direction of leaves of the collimator, or, the translation direction of the block body is perpendicular to the movement direction of the leaves of the collimator; or, the movement direction of the first collimating body is parallel to the movement direction of the leaves of the collimator, or, the movement direction of the first collimating body is perpendicular to the movement direction of the leaves of the collimator. FIG. 8 illustrates an example where the translation direction of the block body is perpendicular to the movement direction of the leaves of the collimator, and the movement direction of the first collimating body is perpendicular to the movement direction of the leaves of the collimator.

The compound collimator provided in the present disclosure forms a radiation field through the collimator 12 and the block 13. For complex large-radiation-field lesions, the radiation beams 10a pass through the collimator channel 12a to form a complex large-radiation-field (conformal radiation field Fc) with the collimator 12 as primarily conforming, and the block 13 mainly shield the leakage radiation outside the radiation field along the edge of the complex large-radiation-field. In the case that a precise small-radiation-field is required for SBRT/SRS treatment or local dose enhancement for tumors, the collimator 12 envelopes to form a quasi-circular channel, and the corresponding first collimating channel 13a on the block 13 moves to the beam center axis, jointly forming a beam channel. The radiation beams 10a form a focused radiation field Ff in response to passing through the collimator channel 12a and the first collimating channel 13a of the block 13.

The compound collimator provided in the present disclosure, through the movement of the block 13, enables the first collimating channel 13a of the block 13 to move to the beam center axis to achieve the focused radiation field Ff by cooperating with the collimator channel 12a, or enables the first collimating channel 13a of the block 13 to deviate from the beam center axis and achieve the conformal radiation field Fc after only passing through the collimator channel 12a. By switching between the conformal radiation field Fc and the focused radiation field Ff, a flexible irradiation scheme is provided, thereby improving the dose distribution effect and enhancing the radiation therapy effect.

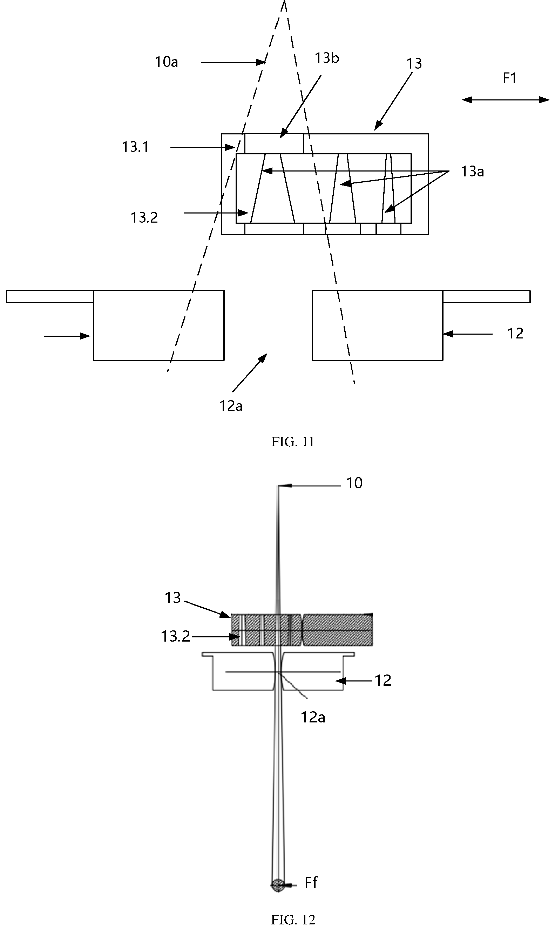

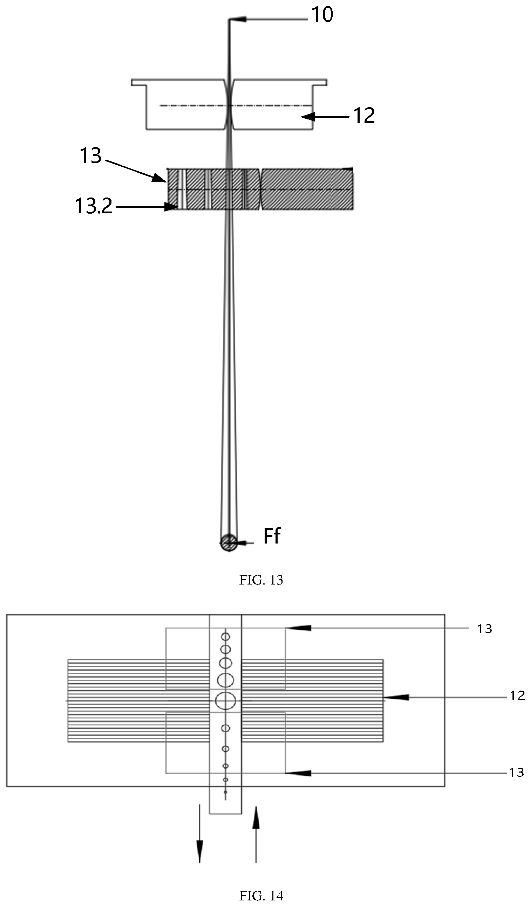

It should be noted that this disclosure does not limit the order in which the radiation beams 10a pass through the collimator 12 and the block 13. In some embodiments, as shown in FIGS. 12 and 13, the block 13 is closer to the radiation source 10, in which case the radiation beams 10a pass through the block 13 first and then the collimator 12. Alternatively, in the case that the collimator is closer to the radiation source, the radiation beams pass through the collimator first and then the block. It should be noted that FIGS. 12 and 13 are rotational cross-sectional views to more clearly illustrate that the order of the collimator and the block is not limited.

In the embodiments provided by the present disclosure, the block includes a block body and a first collimating body disposed on the block body and movable relative to the block body. The block body is configured to drive the first collimating body to translate. A first collimating channel is disposed in the first collimating body, and a block channel coaxially corresponding to the first collimating channel is disposed in the block body. Through translation of the block body and movement of the first collimating body, the first collimating channel and the block channel are caused to deviate from the beam center axis, or the first collimating channel and the block channel are caused to be located on the beam center axis.

In the embodiments of the present disclosure, there are a plurality of implementation methods for making the first collimating channel and the block channel deviate from the beam center axis or for positioning the first collimating channel and the block channel on the beam center axis through the translation of the block body and the movement of the first collimating body. In some embodiments, the movement of the first collimating body includes the rotation of the first collimating body, and/or the translation of the first collimating body. In some embodiments, the first collimating body is a cylinder or a cuboid, with different first collimating channels arranged on it. In some embodiments, the first collimating body switches between the first collimating channels by rotating. In some embodiments, the block is equipped with one block channel or a plurality of block channels. The various motion modes of the block and the first collimating body cooperate with each other to make the first collimating channel and the block channel deviate from the beam center axis, or to position the first collimating channel and the block channel on the beam center axis.

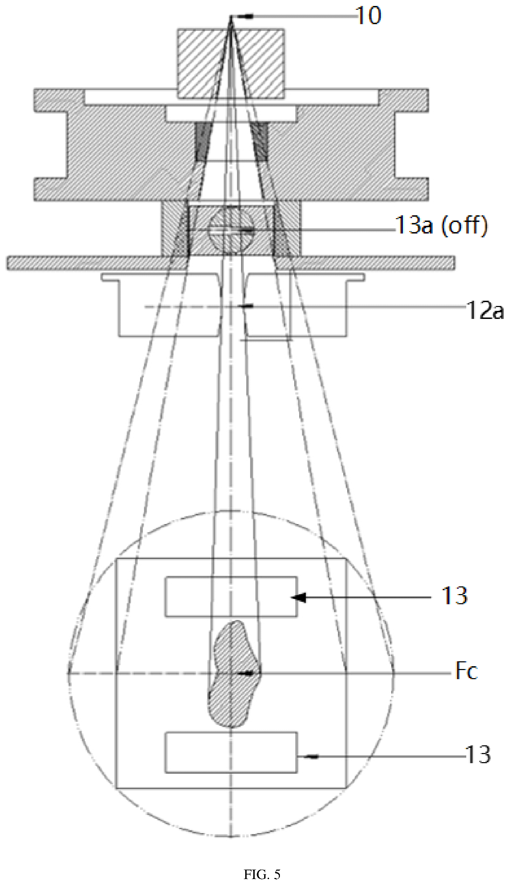

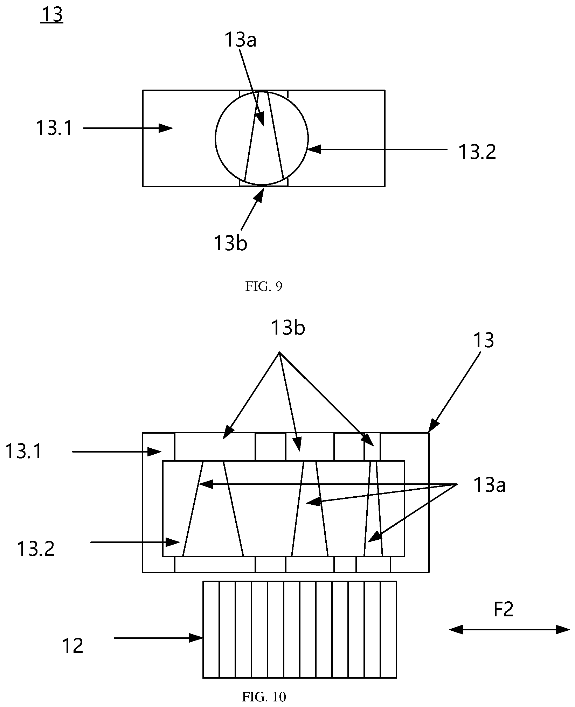

In some embodiments, as shown in FIGS. 7 and 8, the block 13 includes a block body 13.1 and a first collimating body 13.2 disposed on the block body 13.1 and movable relative to the block body 13.1. The block body 13.1 is configured to drive the first collimating body 13.2 to translate along a first direction F1. A first collimating channel 13a is disposed in the first collimating body 13.2, and a block channel 13b for coaxial correspondence with the first collimating channel 13a is disposed in the block body 13.1. In some embodiments, the block channel 13b corresponds to the first collimating channel 13a, and the aperture size of the block channel 13b is larger than the aperture size of the first collimating channel 13a. In some embodiments, as shown in FIG. 9, in the case that the dimensions of the block body 13.1 and the first collimating body 13.2 along the beam center axis are substantially the same, the block channel 13b is substantially close to zero. As shown in FIGS. 4 and 5, the block channel is substantially invisible.

As shown in FIGS. 7 and 8, in some embodiments, the first collimating body is cylindrical, and the first collimating body 13.2 is capable of perform translational and rotational movements along the beam center axis. As shown in FIG. 7 or FIG. 4, in the case that the first collimating body 13.2 rotates such that the block channel 13b aligns with the first collimating channel 13a, the first collimating channel is open. In the case that the first collimating body 13.2 rotates by 90°, as shown in FIG. 5, the first collimating channel is closed, and radiation cannot pass through the first collimating channel 13a. Here, "align" refers to the block channel being coaxial or approximately coaxial with the first collimating channel, allowing the radiation beam to pass through the first collimating channel. In some embodiments, the first collimating channel is made non-coaxial with the block channel through the translation of the first collimating body; and in the case that the first collimating channel is shielded by the block, it achieves closure of the first collimating channel.

In some embodiments, as shown in FIG. 8, the first collimating body 13.2 is provided with a plurality of first collimating channels of different sizes. The first collimating body 13.2 translates along the second direction F2, such that different first collimating channels 13a correspond to the block channel 13b, thereby switching to different first collimating channels of different sizes as needed. In some embodiments, the first collimating body 13.2 translates along the first direction F1, such that different first collimating channels 13a correspond to the block channel 13b. The embodiments of the present disclosure are illustrated only by the example shown in FIG. 8.

In some embodiments, as shown in FIG. 10, the block body 13.1 is provided with a plurality of block channels 13b, and the first collimating body 13.2 is provided with a plurality of first collimating channels 13a, with the plurality of first collimating channels corresponding to the plurality of block channels. By moving the block, different first collimating channels are switched to be positioned on the beam axis, such that the first collimating channels are closed or opened (that is, to make the first collimating channels parallel to or intersect (e.g., perpendicular to) the beam axis) only through the rotation of the first collimating body 13.2.

In some embodiments, the first collimating body is not cylindrical, for example, the first collimating body is rectangular. In some embodiments, the switching of the first collimating channel is achieved through the translation of the first collimating body. Additionally, in some embodiments, the first collimating channel is shielded by the block body through the translation of the first collimating body, thereby closing the first collimating channel.

In some embodiments, the first collimating body has a spherical structure, on which a plurality of first collimating channels passing through the center of the sphere. By rotating, different first collimating channels correspond to the beam center axis.

In some embodiments, as shown in FIGS. 8 to 10, the block moves along the second direction F2, and the first collimating body moves along the second direction F2. The first direction F1 is the direction of motion of the leaf, meaning that the direction of motion of the block is different from that of the leaf. The embodiments of the present disclosure do not limit the relationship between the direction of motion of the block, the direction of motion of the leaf, and the direction of motion of the first collimating body. In some embodiments, the direction of motion of the block is the same as that of the leaf, as shown in FIG. 7, where the block moves along the first direction F1. In some embodiments, the direction of motion of the first collimating body is along the first direction F1 or the second direction F2. In some embodiments, as shown in FIG. 11, the direction of motion of the first collimating body is the first direction F1. The embodiments of the present disclosure are exemplified only by the examples shown in FIGS. 7 to 11 for illustrative purposes.

In the embodiments provided by the present disclosure, the block body 13.1 and the first collimating body 13.2 are arranged along the direction of the beam center axis. A block channel 13b is formed in the block body 13.1, and a first collimating channel 13a is formed in the first collimating body 13.2. The block body 13.1 is translatable relative to the beam center axis, and the first collimating body 13.2 is capable of perform both translational and rotational movements relative to the beam center axis. In some embodiments, the translation of the block body 13.1 drives the first collimating body 13.2 to translate; and in the case that the first collimating body 13.2 moves, the block body 13.1 does not move along with it.

In some embodiments, a plurality of first collimating channels are arranged on the first collimating body, and the first collimating channels have different aperture sizes. In the case that the first collimating channels of different sizes move to the beam center axis, focused radiation fields Ff of different sizes are formed. In some embodiments, the aperture size of the first collimating channel 13a is generally less than 30 cm. In some embodiments, the aperture sizes of the first collimating channels 13a have many options. In some embodiments, the aperture size of the first collimating channel 13a is 1 cm, 3 cm, 5 cm, 7 cm, 10 cm, 12 cm, 16 cm, 18 cm, 20 cm, 25 cm, etc. Those skilled in the art can set the aperture size according to actual needs.

FIG. 12 illustrates an example where the radiation beams 10a sequentially pass through the block channel, the first collimating channel, and the collimator channel. FIG. 13 shows the radiation beams sequentially passing through the collimator channel, the block channel, and the first collimating channel.

In some embodiments, the blocks include two oppositely arranged ones, as shown in FIGS. 12 and 13. The left block 13 participates in forming a radiation field, with its first collimating channel 13a in the open position. The right block 13 does not participate in forming a radiation field, with its first collimating channel 13a in the closed position. In FIGS. 12 and 13, the radiation beams 10a form a focused radiation field Ff in the target volume. FIGS. 12 and 13 are rotational cross-sectional views of FIGS. 4 and 5, to more clearly show the paired blocks.

In some embodiments, a collimator and a block jointly form a radiation field, both of which are independently controlled. The block is usually in pairs, and includes two oppositely arranged block components. At least one of the two block components is provided with a first collimating channel, or, each of the two block components is separately provided with a first collimating channel.

Each block component includes a block body and a first collimating body, the arrangement of which is referred to above and will not be further described. In the case that only one block component is provided with a first collimating channel, the first collimating channel is moved to position it on or off the beam center axis; and in the case that both block components are provided with a first collimating channel, only one block component participates in the radiation field formation, and the first collimating channel of the block component involved in the radiation field formation is located on the beam center axis.

In some embodiments provided by the present disclosure, two block components are respectively provided with a first collimating channel. In some embodiments, as shown in FIG. 14, the first collimating channel of one block component is larger than that of the other block component, so as to distribute a variety of first collimating channels of different sizes on the two block components for selection. That is, the smallest first collimating channel of one block is larger than the largest first collimating channel of the other block, to achieve rapid switching between similar first collimating channels.

In the case that the two block components are separately equipped with a first collimating channel, the first collimating channel of one block component is located on the beam center axis, and the first collimating channel of the other block component intersects with the beam center axis, thereby preventing the radiation beams from leaking through the first collimating channel of the other block component.

In some embodiments provided by the present disclosure, the block component with the first collimating channel on the beam center axis participates in the radiation field formation, and another block component does not participate. To prevent the radiation beams from scattering to the first collimating channel of the block component that does not participate in the radiation field formation, the first collimating channel of the block component that does not participate in the radiation field formation needs to be rotated to intersect with the beam center axis, so as to avoid the radiation passing through the first collimating channel. Preferably, in the case that the first collimating channel of the block component that does not participate in the radiation field formation is rotated to be perpendicular to the beam center axis, the effect of shielding the radiation beam by the block component that does not participate in the radiation field formation is better. In some embodiments, the radiation is avoided from passing through the first collimating channel by moving the block component, or by rotating the first collimating body, thereby achieving the closure of the first collimating channel on the first collimating body.

By mounting the first collimating body on the block body as described above, the first collimating body is moveable relative to the block body to switch the first collimating channel on and off. The entire block moves to position the selected first collimating channel to the beam center axis. In the case that the first collimating body is in the open position, the radiation beams form the desired precise focused radiation field Ff shape on the isocenter plane in response to passing through the collimator channel and the first collimating channel, as shown in FIG. 4. In the case that the first collimating channel moves away from the beam center axis, the first collimating body is in the closed position, and the radiation beams form a conformal radiation field Fc on the isocenter plane in response to passing through the collimator channel. At this time, the block moves outside the beam center axis, mainly shielding against leakage radiation outside the radiation field along the edges of the complex radiation field, as shown in FIG. 5.

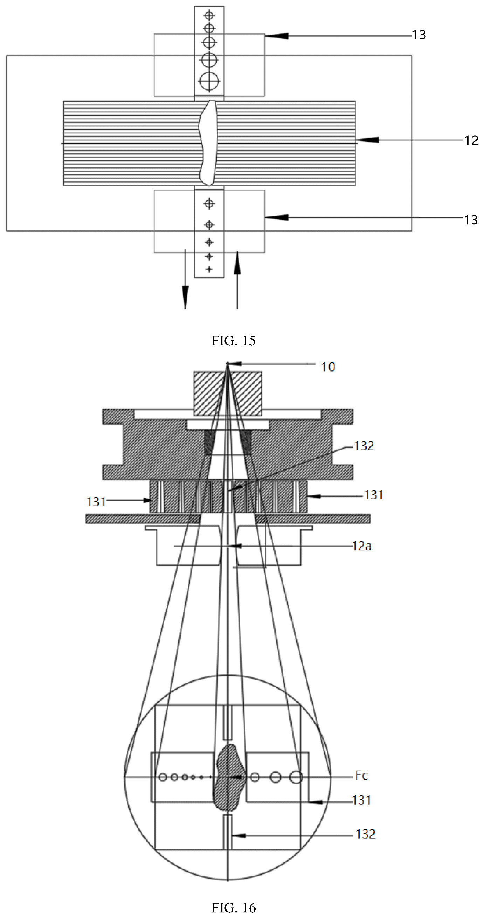

In some embodiments, as shown in FIG. 14, in the case that a focused radiation field Ff is required, the selected first collimating channel is positioned on the beam center axis through the reciprocal motion of the block, and both the block and the collimator block the edge of the precise focused radiation field Ff to reduce the leakage of radiation outside the radiation field. As shown in FIG. 15, in the case that a conformal radiation field Fc is required, the block moves back and forth to the evasive position, and the collimator is configured to shape the radiation field. The block accordingly blocks the edge of the conformal radiation field Fc formed by the collimator along the edge.

In some other embodiments provided in the present disclosure, as shown in FIGS. 16 to 18, the block 13 includes a first block group 131 and a second block group 132 that are orthogonally arranged and translatable. The first block group 131 is formed with a first collimating channel 13a; and, through the translating of the first block group 131, the first collimating channel 13a is made to deviate from or align with the beam center axis. The second block group 132 is configured to block the edge gaps of the collimator channel 12a.

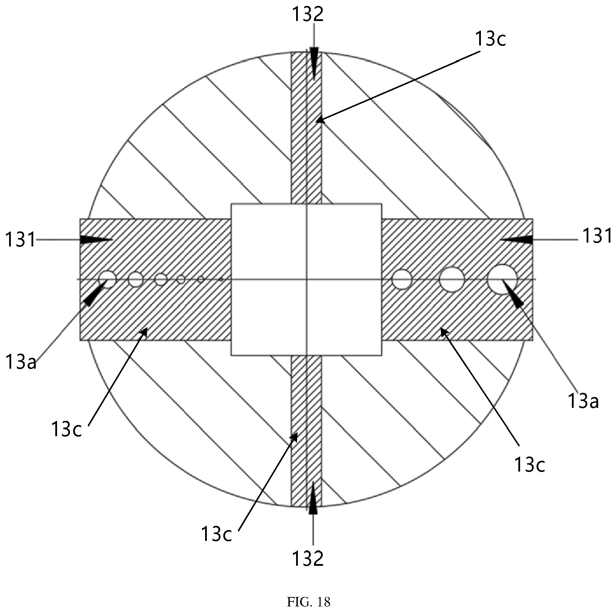

In some embodiments, as shown in FIG. 18, each block group further includes two block bodies 13c arranged relative to the beam center axis. The two block groups move orthogonally, and the two block bodies 13c of each block group are independently controlled to move. In some embodiments, as shown in FIGS. 16 to 18, the translation direction of the first block group 131 is parallel to the movement direction of the leaves of the collimator 12. Alternatively, the translation direction of the second block group 132 is the same as the movement direction of the leaves of the collimator 12.

In some embodiments, in the case that the translation direction of the first block group 131 is parallel to the leaf movement direction of the collimator 12, the translation direction of the second block group 132 is perpendicular to the leaf movement direction of the collimator 12. In the embodiments of the present disclosure, the second block group 132, whose translation direction is perpendicular to the leaf movement direction, has a slightly thinner size, with a thickness smaller than that of the first block group 131 (hereinafter referred to as the thin block group). The second block group 132 is mainly configured to block the leakage rays from the gaps at the ends of the leaves.

The first block group 131, which moves along with the leaf, is pre-equipped with a first collimating channel 13a. The independent movement of the first block group 131 allows the selected first collimating channel 13a to be positioned on the beam center axis. In some embodiments, there are a plurality of first collimating channels 13a, each with different aperture sizes. In the case that the first block group 131 translates, different first collimating channels 13a correspond to the beam center axis.

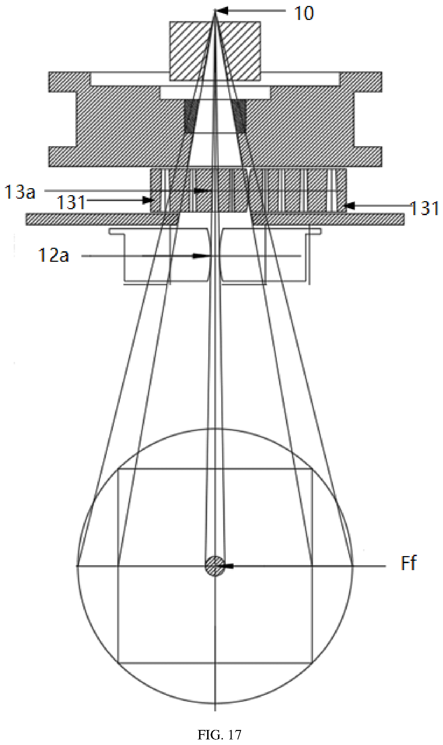

As shown in FIG. 17, in the case that it is necessary to form a precise focused radiation field Ff, the thin block group retreats to the outermost position, and the block group with the first collimating channel moves to position the selected first collimating channel on the beam center axis and enable the selected first collimating channel to coordinate with the collimator; and the leaves of the collimator move to enclose a quasi-circular/conical collimator channel. As shown in FIG. 16, in the case that a conformal radiation field Fc is required, both block groups retreat to the outer side, the first collimating channel deviates from the beam center axis, and the radiation beams pass only through the collimator channel to form a conformal radiation field Fc. At this time, both block groups are configured to block the leakage radiation from the leaves.

In some embodiments, as shown in FIGS. 16 to 18, the first block group 131 and the second block group 132 each include two block bodies, with the two block bodies of the same group being arranged opposite to each other. In some embodiments, the block group with the first collimating channel includes two block bodies, each of which is provided with a plurality of first collimating channels, and the sizes of the plurality of first collimating channels are different. In some embodiments, the maximum aperture size of the first collimating channel on one block body is smaller than the minimum aperture size of the first collimating channel on the other block body. In some embodiments, in the case that the first collimating channel is conical, the conical first collimating channel focuses on the target point.

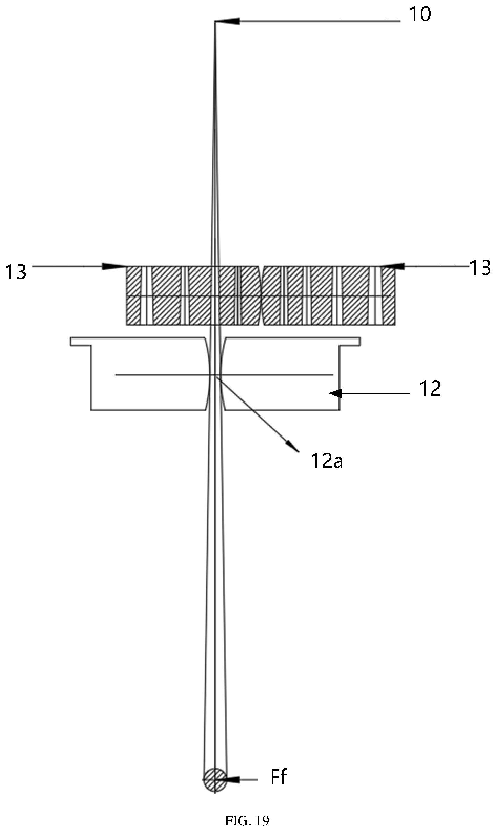

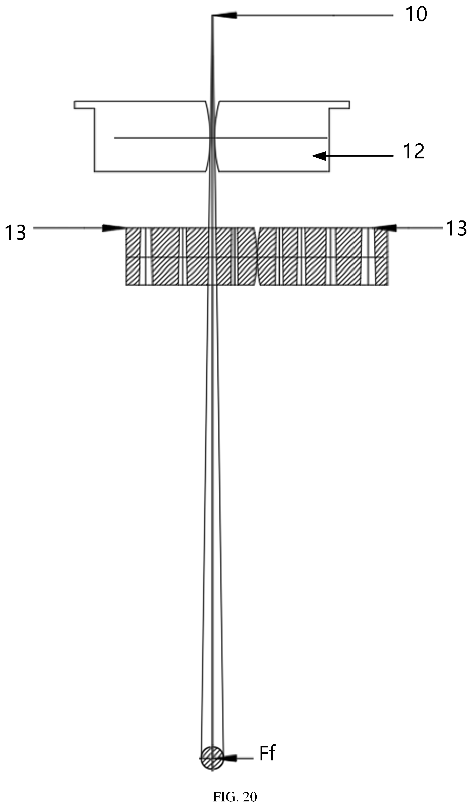

The present disclosure does not impose any restrictions on the order of the positions of the block and collimator. In some embodiments, as shown in FIG. 19, the radiation beams 10a first pass through the first collimating channel 13a of a block 13, and then pass through the collimator channel 12a; or as shown in FIG. 20, the radiation beams 10a first pass through the collimator channel 12a, and then pass through the first collimating channel 13a of a block 13.

In the embodiments of the present disclosure, the block group following the movement of the leaves primarily blocks the leakage radiation between the leaves. A first collimating channel is predetermined on the block group. In the case that the block group is in the non-working position, the first collimating channel is not parallel to the beam direction, the radiation beam is blocked and prevented from directly penetrating, and the enhanced protection against the slight leakage radiation from the leaf gaps is not affected. Only in the case that the first collimating channel is in the working position, the radiation beams are capable of passing through the first collimating channel.

In some embodiments, the first block group 131 and the second block group 132 are located on the same plane, forming a compact structure and a small overall axial dimension. Alternatively, the first block group 131 and the second block group 132 are located on different planes.

Through the above embodiments, the purpose of moving the first collimating channel to the beam center axis or away from the beam center axis is achieved. The present disclosure does not specifically limit the shape of the first collimating channel, which can be a cylindrical channel, a conical channel, or other channels.

Generally, the first collimating channel is a conical channel that constrains the radiation beams generated by the radiation source to a precise shape, typically circular, on the isocenter plane. In the case that the channel is conical, optionally, the cross-sectional size of the end of the conical channel close to the radiation source is smaller than the cross-sectional size of the end of the conical channel away from the radiation source.

In some embodiments, the block channel, the collimator channel, and the first collimating channel are coaxial, and various conical channels during the non-coplanar irradiation focus on the target point (which is an accelerator target point or a gamma knife radiation source). The penumbra is small, the radiation field accuracy is high, the edge of the radiation field is continuous and smooth, and the size is precise.

Through the aforementioned compound collimator, switching between different radiation fields is achieved. In the case that the compound collimator is applied to the radiotherapy system of the present disclosure, the compound collimator is used as a conventional conformal intensity-modulated collimator, or used to form precise radiation field shapes to achieve precise treatment of SRS/SBRT.

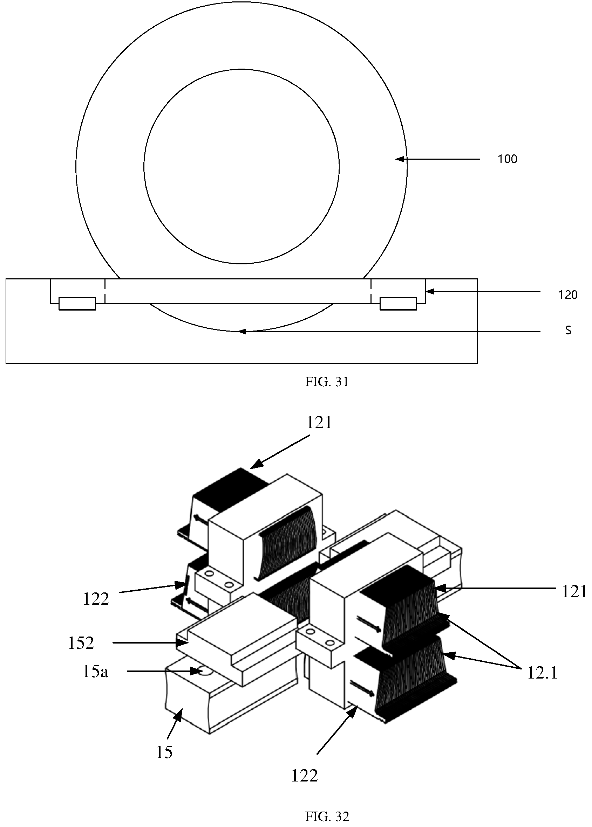

In other embodiments, referring to FIGS. 32 to 34, the present disclosure provides another compound collimator for shaping the radiation beams 10a emitted by a radiation source, including:
a first leaf group 121 and a second leaf group 122 that are arranged sequentially along the direction of the beam center axis, and a second collimating body 15 that is arranged orthogonally to the second leaf group on the same layer as the second leaf group;
wherein the first leaf group 121 and the second leaf group 122 each include a plurality of leaves 12.1, and the movement of the plurality of leaves 12.1 forms collimator channels allowing the radiation beams to pass through; and
the second collimating body 15 is provided with a second collimating channel 15a, and the second collimating body 15 is moveable relative to the beam center axis. In the case that the second collimating body 15 moves to make the collimating channel 15a on the beam center axis, the radiation beams 10a form a focused radiation field in the target volume in response to passing through the collimator channel formed by the first leaf group 121 and the second collimating channel 15a. In the case that the second collimating body 15 moves to make the second collimating channel 15a deviate from the beam center axis, the radiation beams 10a form a conformal radiation field in the target volume in response to passing through the collimator channel formed by the first leaf group 121 and/or the collimator channel formed by the second leaf group 122.

Similar to the previous embodiments, the aforementioned "the second collimating body 15 moves to make the collimating channel 15a on the beam center axis" refers to the movement of the second collimating body 15 enabling the beam center axis to pass through the collimating channel 15a. The aforementioned "the second collimating body 15 moves to make the second collimating channel 15a deviate from the beam center axis" refers to the movement of the second collimating body making the second collimating body move to an evasive position where the second collimating body does not obstruct the radiation beams from passing through the collimator channel.

The first leaf group 121 and the second leaf group 122 are arranged in two layers along the beam center axis, with each group including a plurality of leaves 12.1. The movement of the plurality of leaves in the first leaf group 121 or the second leaf group 122 forms collimator channels of different shapes that allow the radiation beams to pass through. In some embodiments, the leaves 12.1 of the first leaf group 121 and the leaves 12.1 of the second leaf group 122 are arranged to overlap with each other, with the leaves 12.1 of the second leaf group 122 blocking the gaps between the leaves 12.1 of the first leaf group 121.

In some embodiments, as shown in FIG. 32, the first leaf group 121 and the second leaf group 122 are respectively arranged in two boxes, correspondingly, with the two boxes being located on the upper and lower layers respectively. In some embodiments, the two boxes are separately fixed or connected and fixed. Alternatively, the first leaf group 121 and the second leaf group 122 are arranged in the same box, with the first leaf group 121 and the second leaf group 122 being located on the upper and lower layers within the same box.

The movement of the leaves 12.1 forms collimator channels, in other words, the first leaf group 121 forms a collimator channel, and the second leaf group 122 also forms a collimator channel, the collimator channel allowing the radiation beam 10a to pass through.

In some embodiments, the first leaf group 121 is arranged close to the radiation source 10, and the second leaf group 122 is arranged on the side away from the radiation source 10. That is to say, the radiation beams 10a emitted from the radiation source 10 first pass through the collimator channel formed by the first leaf group 121, and then pass through the collimator channel formed by the second leaf group 122. The present disclosure takes the example where the first leaf group 121 is arranged close to the radiation source 10, and the second leaf group 122 is arranged on the side away from the radiation source 10.

Of course, it is also possible to arrange the second leaf group 122 close to the radiation source 10, and the first leaf group 121 on the side away from the radiation source 10. In this case, the radiation beams 10a emitted from the radiation source 10 first pass through the collimator channel formed by the second leaf group 122, and then pass through the collimator channel formed by the first leaf group 121.

The compound collimator provided in the present disclosure further includes a second collimating body 15, which is located on the same layer as the second leaf group 122 and is arranged orthogonally to the second leaf group 122. The second collimating body 15 is provided with a second collimating channel 15a, and the second collimating body 15 is moveable relative to the beam center axis to make the second collimating channel 15a parallel to the beam center axis to allow the beam center axis to pass through.

In the case that the second collimating body 15 moves, the second collimating channel 15a is made along the beam center axis in some embodiments. At this time, the second leaf group 122 retreats away from the beam center axis to avoid the movement path of the second collimating body 15. The radiation beams 10a pass through the collimator channel of the first leaf group 121 and the second collimating channel 15a, forming a focused radiation field in the target volume. After the second leaf group 122 retreats from the beam center axis, the second leaf group 122 moves closer to the beam center axis again in some embodiments. The ends of the leaves 12.1 of the second leaf group 122 fit both sides of the second collimating body 15 to block leakage of the surrounding radiation beams 10a. The focused radiation field formed in this way is suitable for particularly small lesion volumes with many surrounding organs at risk.

In the case that the second collimating body 15 moves to a position where the second collimating channel 15a deviates from the beam center axis, in some embodiments, both the first leaf group 121 and the second leaf group 122 move to the beam center axis. The radiation beams 10a pass through the collimator channel formed by the first leaf group 121 and the collimator channel formed by the second leaf group 122, forming a conformal radiation field in the target volume with higher conformal accuracy.

In some embodiments, either the first leaf group 121 or the second leaf group 122 moves to the beam center axis. At this time, the radiation beams 10a form a conformal radiation field in the target volume in response to passing through the collimator channel formed by the leaf group that has moved to the beam center axis, that is, passing through the collimator channel formed by the first leaf group 121 or the collimator channel formed by the second leaf group 122. The conformal radiation field is suitable for complex large-radiation-field lesions. In some embodiments, the other leaf group that does not move to the beam center axis approaches the beam center axis to shield the leakage radiation outside the radiation field along the edge of the complex large-radiation-field lesion. Alternatively, in some embodiments, a conformal radiation field is formed by one leaf group, and intensity modulation is achieved through the movement of the leaves 12.1 of the other leaf group.

Therefore, the compound collimator provided in the embodiments of the present disclosure includes a first leaf group 121 and a second leaf group 122 sequentially arranged along the beam center axis direction. The first leaf group 121 and the second leaf group 122 each include a plurality of leaves 12.1, and the movement of the plurality of leaves 12.1 forms a collimator channel allowing the radiation beams 10a to pass through. A second collimating body 15 is further orthogonally disposed in the same layer as the second leaf group 122, and the second collimating body 15 is equipped with a second collimating channel 15a. The second collimating body 15 is moveable relative to the beam center axis. In the case that the second collimating body 15 moves to a position where the second collimating channel 15a is aligned with the beam center axis, the radiation beams 10a pass through the collimator channel formed by the first leaf group 121 and the second collimating channel 15a to form a focused radiation field in the target volume, which is suitable for the treatment of particularly small lesion volumes with many surrounding organs at risk. The second leaf group 122 approaches the beam center axis, and the ends of the leaves 12.1 of the second leaf group 122 fit against both sides of the second collimating body 15 to block leakage of the surrounding radiation beams 10a. In the case that the second collimating body 15 moves such that the second collimating channel 15a deviates from the beam center axis, the radiation beams 10a pass through the collimator channel formed by the first leaf group 121 and/or the collimator channel formed by the second leaf group 122 to form a conformal radiation field in the target volume, which is suitable for the treatment of complex large-radiation-field lesions. At this time, in the case that only one leaf group is located at the beam center axis and the radiation beams 10a pass through its collimator channel, the other leaf group that has not moved to the beam center axis acts to block leakage of radiation outside the radiation field.

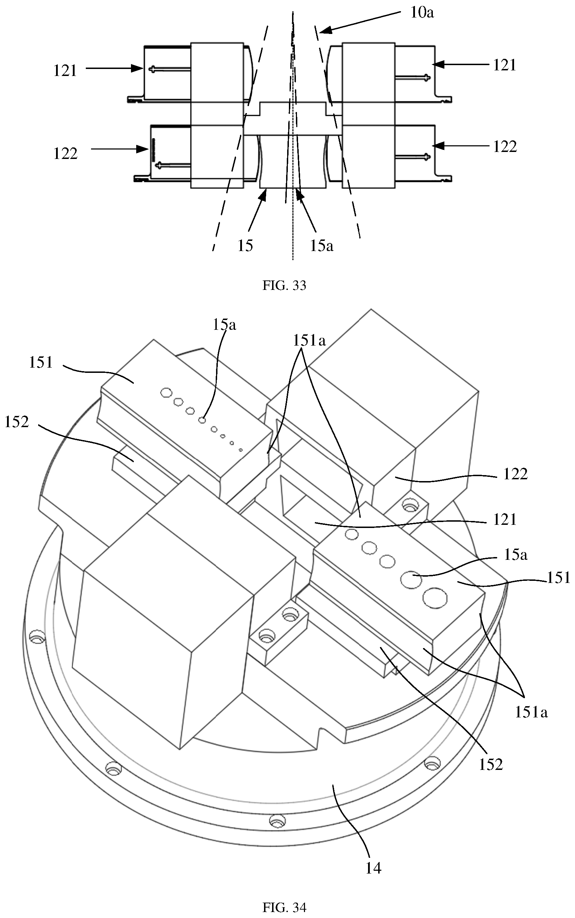

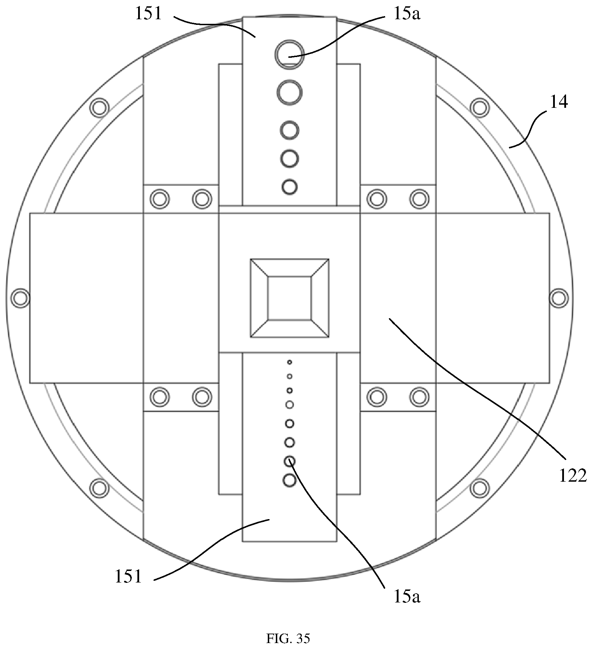

In some embodiments, as shown in FIGS. 34 to 35, in the embodiments provided by the present disclosure, the second collimating body 15 is provided with a plurality of second collimating channels 15a, each with a different aperture size. In the case that the second collimating channels 15a of different sizes move to the beam center axis, they can form focused radiation fields of varying sizes. In some embodiments, the aperture size of the second collimating channel 15a is generally less than 30 cm; in other embodiments, the aperture sizes of the second collimating channels 15a have many options; and in some embodiments, the sizes include 1 cm, 3 cm, 5 cm, 7 cm, 10 cm, 12 cm, 16 cm, 18 cm, 20 cm, 25 cm, and the like. Those skilled in the art can set the aperture size according to actual needs.

The second collimating body 15 includes two oppositely arranged collimating sub-bodies 151, wherein at least one of the two collimating sub-bodies 151 is provided with a second collimating channel 15a, or the two collimating sub-bodies 151 are each provided with a second collimating channel 15a. In some embodiments, as shown in FIG. 34, the embodiments and accompanying drawings of the present disclosure illustrate an example where the two second collimating sub-bodies 151 are each provided with a second collimating channel 15a.

In some embodiments, as shown in FIG. 34, a plurality of second collimating channels 15a are arranged in each of the two opposite collimating sub-bodies 151. The second collimating channel 15a of one collimating sub-body 151 is larger than that of the other collimating sub-body 151, meaning that the smallest second collimating channel 15a of one collimating sub-body 151 is larger than the largest second collimating channel 15a of the other collimating sub-body 151. This allows for a variety of second collimating channels 15a of different sizes to be distributed on the two collimating sub-bodies 151 for selection, enabling rapid switching between similar second collimating channels 15a.

In some embodiments, as shown in FIG. 34, two opposite collimating sub-bodies 151 are mounted on a pair of guide rails 152, and are capable of advancing and retreating under the drive of a driving device. Their movement direction is perpendicular to the movement direction of the leaves 12.1 of the second leaf group 122 in the same layer.

In some embodiments, the second collimating channel 15a includes a cylindrical channel or a conical channel. The embodiments of the present disclosure take the second collimating channel 15a as a conical channel as an example for illustrative purposes. The cross-sectional size of the end of the conical channel close to the radiation source 10 is smaller than that of the end of the conical channel away from the radiation source 10.

In the embodiments provided in the present disclosure, the compound collimator consists of two layers of leaf groups and a second collimating body 15 with a conical second collimating channel 15a. The second collimating body 15 and the leaf group at a layer close to the patient in the two layers of leaf groups are located on the same plane, with their movement directions orthogonal to each other. In the case that it is necessary to form a focused radiation field, the leaves 12.1 of the second leaf group 122 retreat to avoid the movement path of the second collimating body 15. After the selected second collimating channel 15a in the second collimating body 15 moves onto the beam center axis, the leaves 12.1 of the second leaf group 122 converge towards the beam center axis, with the ends of the leaves 12.1 of the second leaf group 122 fitting both sides of the second collimating body 15 to block leakage of the surrounding radiation beams 10a. The leaf group closer to the target converges towards the beam center axis, forming a quasi-circular collimator channel. The actions of the two layers of leaf groups are synchronized in some embodiments.

In the case that a conformal radiation field needs to be formed by a leaf group, the second collimating body 15 moves away from the beam center axis to both sides, avoiding the movement path of the leaf group (i.e., moving to the evasive position). The leaf group forms the required conformal radiation field in a conventional manner. That is, the radiation beams 10a form a conformal radiation field in the target volume in response to passing through the collimator channels formed by the first leaf group 121 and/or the second leaf group 122. In some embodiments, the radiation beams 10a form a conformal radiation field in the target volume only in response to passing through the collimator channel formed by the first leaf group 121 or the second leaf group 122. Alternatively, the radiation beams 10a form a conformal radiation field in the target volume in response to passing through both the collimator channels formed by the first leaf group 121 and the second leaf group 122, i.e., the first leaf group 121 and the second leaf group 122 jointly form a conformal radiation field.

In order to reduce the size of the second collimating body 15 and minimize the leakage radiation outside the second collimating channel 15a, in some embodiments, the side wall of the second collimating body 15 in the present disclosure, which is configured to fit against the ends of the leaves 12.1, is a concave side wall 151a (as shown in FIG. 34) to adapt to the shape of the ends of the leaves 12.1.

In some embodiments, the two sides of the second collimating body 15 are concave side walls 151a that fit with the ends of the leaves 12.1 of the second leaf group 122. The leaves 12.1 of the second leaf group 122 closely fit with the second collimating body 15. For example, in the case that the second collimating body 15 moves to make the second collimating channel 15a be located on the beam center axis, the radiation beams 10a form a focused radiation field in the target volume in response to passing through the collimator channel of the first leaf group 121 and then the second collimating channel 15a. The second leaf group 122 approaches the beam center axis, and the ends of the leaves 12.1 of the second leaf group 122 fit closely with the two sides of the second collimating body 15. The concave side walls 151a of the second collimating body 15 enhance the tightness of their fit, better blocking the leakage of surrounding radiation beams 10a. FIG. 33 shows the closely fitting state of the leaf group close to the patient and the second collimating body 15.

Through the aforementioned compound collimator, switching between different radiation fields is achieved. In the case that the compound collimator is applied to the radiotherapy system of the present disclosure, it can be used for stereotactic and conformal intensity-modulated integrated radiotherapy. A single accelerator X-ray source is used as the radiation source 10 to carry out stereotactic and conformal intensity-modulated treatment in a single-head system. Patients undergo one-time positioning, with precise division of the lesion, and targeted treatment using stereotactic and conformal intensity-modulated methods to improve adaptability and significantly enhance treatment efficiency, providing a guarantee for improving the cure rate.

Through the coordinated movement of the gantry and the treatment couch, a wide range of incident angles are provided, enabling stereotactic targeting of focal spots and stereotactic targeting of complex fields with leaf 12.1.

The embodiments of the present disclosure further provide a radiotherapy system, including a treatment couch, a gantry, and a treatment head. The treatment head is arranged on the gantry, and the treatment couch is configured to carry a patient and move the patient for treatment. The treatment head includes a radiation source and any compound collimator provided in the embodiments of the present disclosure, which are arranged in sequence. The compound collimator constrains the radiation beams emitted by the radiation source to a predetermined radiation field in the target volume.

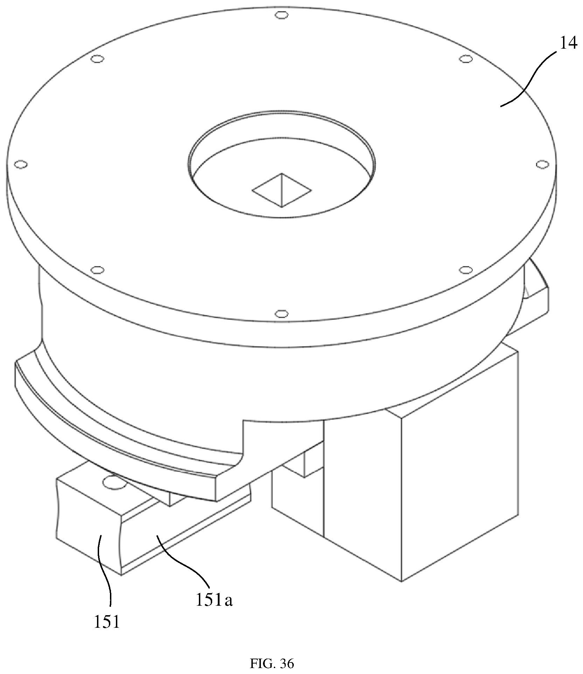

In some embodiments, the radiotherapy system further includes a rotational disc 14, as shown in FIGS. 34 and 36. The compound collimator is disposed on the rotational disc 14, and the rotational disc 14 drives the compound collimator to rotate, such that the two sets of leaf groups and the second collimating body 15 have different angles relative to the radiation source 10, thereby forming different edge positions of the radiation field.

In the case that the compound collimator rotates to different angles, the position of the radiation field formed in the target volume after the radiation beams 10a pass through remains largely unchanged. However, the edges of the formed radiation field change as the compound collimator rotates, to accommodate different lesion shape requirements.

With the aforementioned settings, a single radiation source 10 produces one point source. Through the compound collimator, the focused irradiation and conformal intensity-modulated irradiation are switched for the radiation field. Stereotactic and rotational intensity-modulated treatments are performed on the same device, expanding the treatment range and enhancing the precision of treatment. The coordinated movement of the gantry and treatment couch provides a wide range of incident angles, enabling stereotactic targeting of focused holes and complex radiation fields with collimators. Compared to gamma ray stereotactic devices, the present disclosure has a higher dose rate, smaller penumbra, shorter treatment time, and improved treatment accuracy.

As mentioned earlier, the movement of the second collimating body 15 positions the second collimating channel 15a either on or away from the beam center axis. In some embodiments, referring to FIG. 34, the radiotherapy system further includes a guide rail 152, and the second collimating body 15 is disposed on the guide rail 152 for movement of the second collimating body 15.

In some embodiments of the present disclosure, by relative deflection between the treatment couch and the gantry, different angles are formed between the treatment couch and the gantry, such that the radiation beams are irradiated from different angles, thereby achieving non-coplanar irradiation.

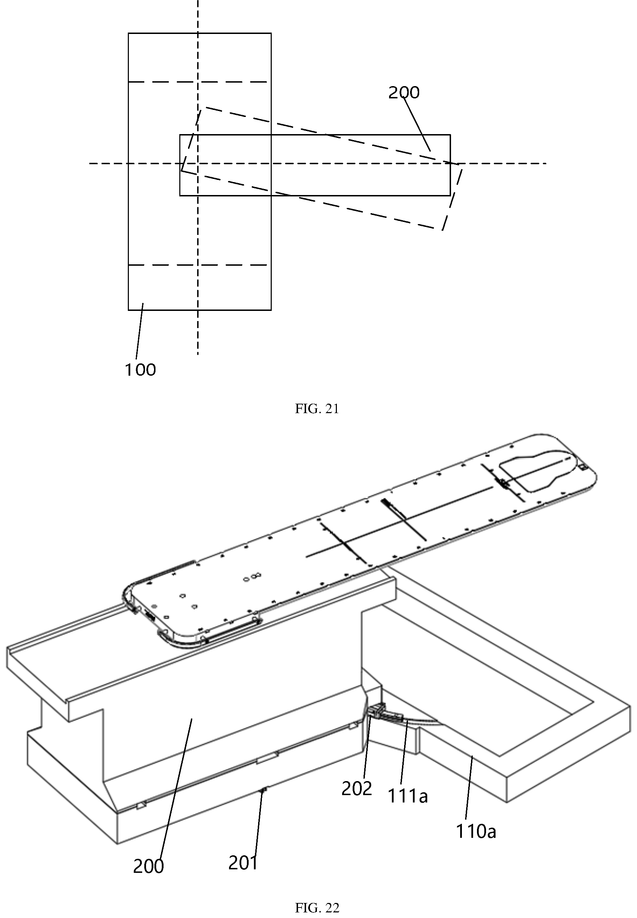

In some embodiments of the present disclosure, as shown in FIG. 21 (which is a top-down view), the gantry 100 and the treatment couch 200 form different angles by deflecting the treatment couch 200. In some embodiments, the gantry 100 rotates around the gantry rotation axis 100b, and the treatment couch 200 is arranged to swing in a horizontal plane, such that the angle between the treatment couch 200 and the gantry 100 varies.

It should be noted that the term "swing in the horizontal plane" mentioned in this disclosure refers to the swing of an object within the horizontal plane relative to an axis perpendicular to the horizontal plane. The term "rotate in the horizontal plane" mentioned in this disclosure refers to the rotation of an object within the horizontal plane with an axis perpendicular to the horizontal plane as the axis of rotation. In the present disclosure, the axis perpendicular to the horizontal plane is referred to as the first axis, and subsequent references to "swing (rotation) in the horizontal plane" also refer to swing (rotation) with the first axis as the axis of rotation.

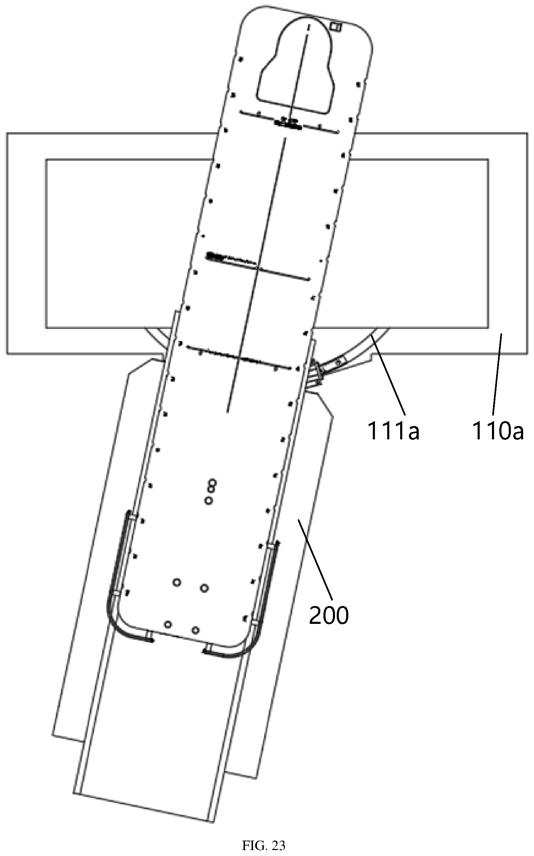

Specifically, referring to FIGS. 22 and 23, in the radiotherapy system provided in the embodiments of the present disclosure, the bottom of the treatment couch 200 is configured to be connected to the third arc-shaped guide rail 111a; and the treatment couch 200 includes a first treatment couch driving device and a second treatment couch driving device. The first treatment couch driving device is configured to drive the treatment couch 200 to move into the treatment space 100a, and the second treatment couch driving device is configured to drive the treatment couch 200 to move along the third arc-shaped guide rail 111a, such that the treatment couch 200 is capable of rotating and swinging in the horizontal plane.

Driven by the first treatment couch driving device, the treatment couch 200 can enter the treatment space 100a along the direction of the gantry rotation axis 100b of the gantry 100. Driven by the second treatment couch driving device, the treatment couch 200 can rotate and swing along the third arc-shaped guide rail 111a in the horizontal plane, forming different angles with the gantry 100, such that the radiation beams 10a can be emitted from different angles to achieve non-coplanar irradiation. In some embodiments, in the case that the angle formed by the treatment couch 200 and the gantry 100 is 0°, it indicates that the axis of the treatment couch 200 is parallel to the gantry rotation axis 100b. It should be noted that the angle formed by the treatment couch 200 and the gantry 100 refers to the angle between the longitudinal axis of the treatment couch and the gantry rotation axis of the gantry.

In some embodiments, the center of the third arc-shaped guide rail 111a is coaxial with the isocenter O of the radiotherapy system. That is, both the center of the third arc-shaped guide rail 111a and the isocenter O of the radiotherapy system are located on the rotational axis (first axis) of the third arc-shaped guide rail 111a, that is, the first axis passes through the center of the third arc-shaped guide rail 111a and the isocenter O of the radiotherapy system.

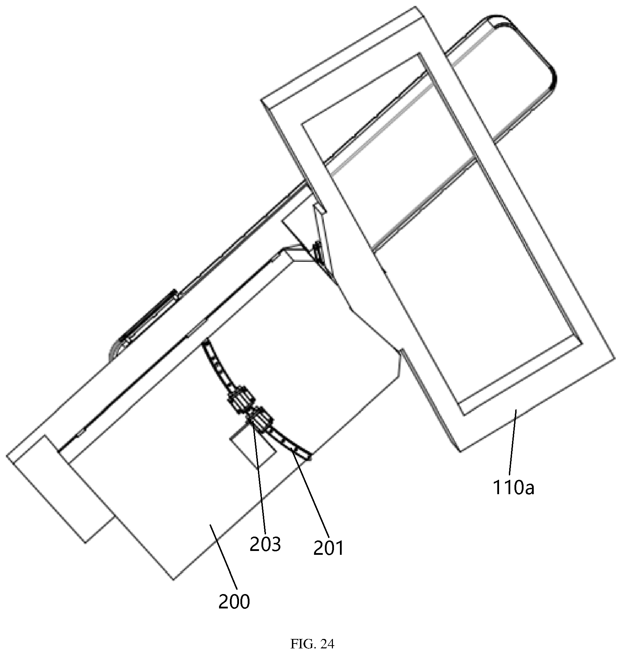

In another embodiment provided by the present disclosure, as shown in FIG. 24, to ensure the support stability of the treatment couch 200, a fourth arc-shaped guide rail 201 is further arranged at the bottom of the treatment couch 200. The two sets of arc-shaped guide rails are concentric but have different radii. In some embodiments, the third arc-shaped guide rail 111a is mounted on the base 110a, and the gantry 100 is positioned on the base 110a to achieve a more compact structure and ensure accuracy.

In the case that the treatment couch 200 is connected to two sets of arc guide rails, the connection with the arc guide rails is achieved through slide blocks, respectively. In some embodiments, the slide block is connected to the treatment couch 200, or the guide rail is connected to the treatment couch 200, which is not limited in the present disclosure. In some embodiments, a first slide block 202 is arranged at the bottom of the treatment couch 200, and the treatment couch 200 is connected to the third arc-shaped guide rail 111a through the first slide block 202. A second slide block 203 is fixed on the ground, and the treatment couch 200 is connected to the second slide block 203 via the fourth arc-shaped guide rail 201. A gap is formed between the bottom of the treatment couch 200 and the ground, and the treatment couch 200 achieves a support function on the ground through the second slide block 203 and the fourth arc-shaped guide rail 201.

Thus, the treatment couch 200 is supported by the arc-shaped guide rail and rotates around the first axis passing through the isocenter to form non-coplanar irradiation. The guide rail of the third arc-shaped guide rail 111a is installed on the base 110a, and the first slide block 202 moves along with the treatment couch 200. In some embodiments, to enhance the support stability of the treatment couch 200, a fourth arc-shaped guide rail 201 is arranged at the bottom of the treatment couch 200, with its guide rail installed at the bottom of the treatment couch 200. The second slide block 203 is connected to the ground via a support frame. The processing of the installation and positioning part of the arc-shaped guide rail on the base 110a is based on the isocenter O point, thereby ensuring the reliability of accuracy.

As shown in FIGS. 23 to 25, an outer cover 101 is integrally provided on the gantry 100 and the base 110a. Both the base 110a and the gantry 100 are located within the outer cover 101, which forms a central opening that matches the treatment space 100a. The third arc-shaped guide rail 111a is located outside the outer cover 101. In this way, the gantry 100 is capable of rotating at high speed within the enclosed structure, reducing the risk of collision with the patient, enhancing the treatment experience, and improving the efficiency and safety of non-coplanar irradiation.

Using a closed design, the moving parts are enclosed within the structure. During the treatment process, real-time monitoring of the target volume can be conducted. After adjusting the non-coplanar angle, during the irradiation process, the patient remains stationary relative to the structure, eliminating the risk of abrasion and collision, thereby enhancing the precision and safety of the treatment.

The present disclosure provides a radiotherapy system that achieves non-coplanar irradiation by swinging the treatment couch, offering a more flexible radiation field arrangement scheme for clinical treatment. The treating physician can adopt a more flexible irradiation method based on the patient's lesion, achieving a better dose distribution. Furthermore, during non-coplanar irradiation, there is no risk of collision, and the gantry can rotate at high speed, enhancing treatment efficiency and safety. Additionally, as mentioned earlier, during the irradiation process, images can be acquired through an image guidance system for patient positioning or real-time monitoring, ensuring treatment efficacy.

The aforementioned method achieves different angles between the treatment couch and the gantry by swinging the treatment couch. In this scenario, after the patient is positioned on the treatment couch, the treatment couch needs to move, thereby resulting in a risk of target volume variation. Furthermore, frequently moving the treatment couch during treatment to change non-coplanar angles can negatively impact the patient's treatment experience.

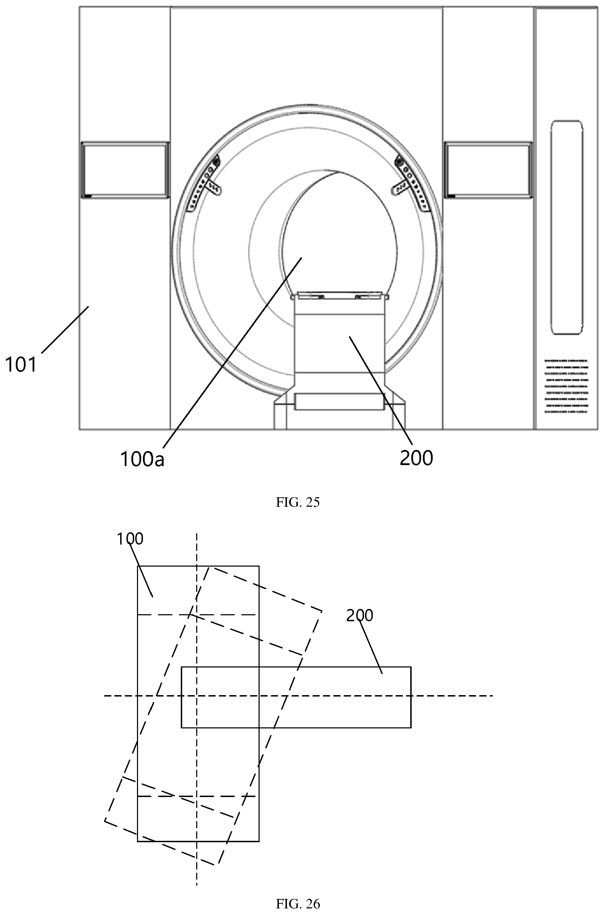

Therefore, in another embodiment of the present disclosure, referring to FIGS. 26 to 29 (FIG. 26 is a top view), the gantry 100 and the treatment couch 200 form different angles through the swing deflection of the gantry 100. After patient positioning, non-coplanar irradiation and focused irradiation are achieved through the swing of the gantry 100, without requiring patient movement, achieving high positioning accuracy and providing a good treatment experience for patients. There are no protrusions inside the gantry 100, eliminating the risk of patient compression and improving the efficiency of non-coplanar irradiation.

During the treatment process, the non-coplanar motion of the gantry 100 can be automatically adjusted, allowing for continuous dynamic adjustments throughout the treatment. Based on the tissue distribution around the target volume, the non-coplanar path can be adjusted to achieve a more optimal dose distribution in the target volume.

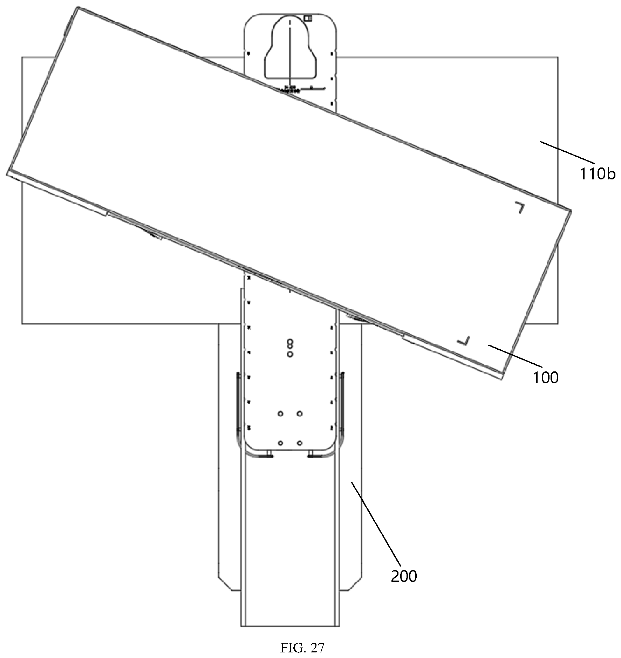

In some embodiments, referring to FIGS. 27 to 31, in the radiotherapy system provided in the embodiments of the present disclosure, the treatment couch 200 is configured to carry a patient and move the patient to a predetermined position. The system further includes a first gantry driving device and a second gantry driving device 120. The first gantry driving device is configured to drive the gantry 100 to rotate about the gantry rotation axis 100b, and the second gantry driving device 120 is configured to drive the gantry 100 to rotate about an axis passing through the isocenter O in a horizontal plane (i.e., rotate about the first axis as the rotation axis), such that the angle between the treatment couch 200 and the gantry 100 is different. The second gantry driving device 120 is higher than the lowest point S of the gantry (the lowest point of the rotation of the gantry 100), as shown in FIG. 31. In some embodiments, the second gantry driving device 120 includes a plurality of different driving components, and the second gantry driving device 120 being higher than the lowest point S of the gantry includes cases that any one or any a plurality of driving components of the second gantry driving device 120 is higher than the lowest point S of the gantry. In some embodiments, the second gantry driving device 120 includes a guide rail, a slide block, and a motor. It is possible for the guide rail to be higher than the lowest point S of the gantry, and the motor is arranged at any position. Alternatively, it is also possible for the guide rail, slide block, and motor to all be higher than the lowest point S of the gantry.

In some embodiments, as shown in FIG. 27, the radiotherapy system further includes a base 110b, which is fixed on the ground or floor. The gantry 100 is arranged on the base 110b and is rotatably connected to the base 110b via a second gantry driving device 120, such that the gantry 100 can rotate and swing relative to the treatment couch 200 in a horizontal plane.

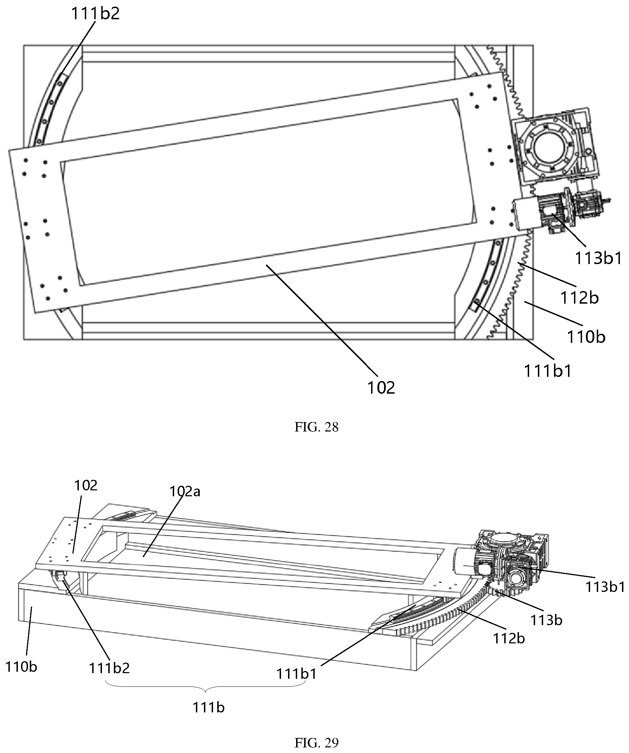

As shown in FIGS. 28 to 30, similar to the swing structure of the treatment couch 200, the gantry 100 achieves swinging through guide rails and slide blocks. In some embodiments, the second gantry driving device 120 includes an arc-shaped guide rail 111b, a slide block, and a drive; wherein the arc-shaped guide rail 111b is arranged on the base 110b, the slide block is arranged on the gantry 100, and the drive is configured to drive the slide block to move along the arc-shaped guide rail 111b, such that the gantry 100 is capable of rotating and swinging relative to the treatment couch 200 in a horizontal plane. The center of the arc-shaped guide rail 111b is coaxial with the isocenter O of the radiotherapy system. That is to say, the first axis is the rotational axis of the arc-shaped guide rail 111b, and the first axis passes through the isocenter O of the radiotherapy system. Here, "the first axis is the rotational axis of the arc-shaped guide rail 111b" means that the arc-shaped guide rail 111b extends in an arc shape with the first axis as the central rotational axis. In some embodiments, the arc-shaped guide rail 111b is arranged on the gantry 100, and the slide block is arranged on the ground. The present disclosure does not limit this, and only uses the illustrations as examples for explanation.

In some embodiments, the arc-shaped guide rail 111b includes a first arc-shaped guide rail 111b1 and a second arc-shaped guide rail 111b2 that are oppositely arranged, and the first arc-shaped guide rail 111b1 and the second arc-shaped guide rail 111b2 are coaxial. Here, "coaxial" refers to the coincidence of the axes of the first arc-shaped guide rail 111b1 and the second arc-shaped guide rail 111b2, where the axes are rotation axes and both are the first axis. In some embodiments, the first arc-shaped guide rail 111b1 and the second arc-shaped guide rail 111b2 are located on both sides of the gantry 100, respectively. In some embodiments, the first arc-shaped guide rail 111b1 and the second arc-shaped guide rail 111b2 are located on both sides of the axis of the gantry 100, respectively, to support the rotation of the gantry 100 from both sides. Correspondingly, the sliding includes a first guide rail slide block and a second guide rail slide block, which are separately connected to the gantry 100; alternatively, the first guide rail slide block and the second guide rail slide block are connected to a connecting piece, and the connecting piece is connected to the gantry 100.

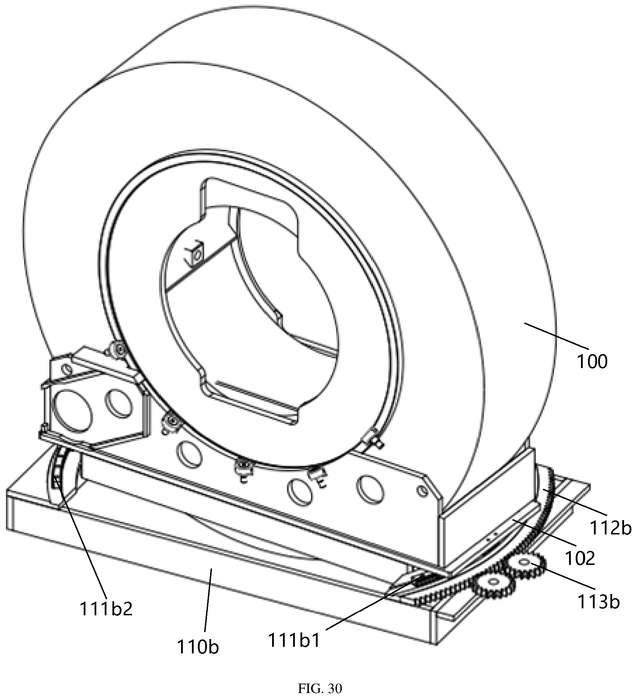

In some embodiments, as shown in FIG. 29, the connecting piece is a tray 102, with the gantry 100 fixed on the tray 102, and the gantry 100 is connected to the base 110b via the tray 102. The bottom of the gantry 100 extends downward through a rectangular hole 102a on the tray 102, such that the arc-shaped guide rail 111b or slide block is higher than the lowest point S of the gantry. In other words, the second gantry driving device 120 is higher than the lowest point S of the gantry, as shown in FIGS. 30 and 31.

In some embodiments, the first arc-shaped guide rail 111b1 and the first guide rail slide block (located on the first arc-shaped guide rail 111b1 and connected to the tray 102) are driven by a drive, which in turn drives the second guide rail slide block (located on the second arc-shaped guide rail 111b2 and connected to the tray 102) to move along the second arc-shaped guide rail 111b2. That is, the first guide rail slide block moves along the first arc-shaped guide rail 111b1 under the driving of the drive, and drives the second guide rail slide block to move along the second arc-shaped guide rail. In this way, in the case that the gantry 100 swings, the first arc-shaped guide rail 111b1 and the second arc-shaped guide rail 111b2 guide the movement of the gantry, which increases the stability of the gantry 100 during swinging.

The drive provides the power for the swinging motion of the gantry 100. In some embodiments of the present disclosure, the drive is a gear drive, including a motor 113b1, a gear 113b, and a gear ring 112b. The gear ring 112b is coaxial with the first arc-shaped guide rail 111b1, and the motor 113b1 drives the gear 113b to move along the gear ring 112b through a steering gear. Here, "coaxial" also refers to the coincidence of the rotational axes of the gear ring and the first arc-shaped guide rail, both being the first axis.

Thus, the arc-shaped guide rail 111b is installed on the base 110b, with its rotational axis being the swinging rotational axis of the gantry 100, which is also the aforementioned first axis. The gantry 100 is connected to the arc-shaped guide rail 111b via a slide block. The base 110b is equipped with a gear ring 112b, whose rotational center is coaxial with the arc-shaped guide rail 111b. The gantry 100 is equipped with a drive, and the gear 113b at the output end of the drive meshes with the aforementioned gear ring 112b. The drive drives the gantry 100 to swing and rotate about the isocenter O by driving the meshing motion of the gear 113b and the gear ring 112b, such that the treatment couch 200 and the gantry 100 form different angles, allowing the radiation beams 10a to be emitted from different angles.

In some embodiments, a positioning pin column is arranged at the bottom of the gantry 100, and the base 110b is connected to the bottom of the gantry 100 through the positioning pin column. The geometric center of the distribution of the positioning pin columns is located on the rotational axis of the base 110b, thereby achieving coincidence between the beam axis of the gantry 100 and the rotational axis of the base 110b. The gantry 100 swings around the central axis of the beam to achieve non-coplanar irradiation. Of course, in the case that the gantry 100 swings in the horizontal plane to form an angle of 90° with the treatment couch 200, coplanar irradiation can be achieved.

In addition, an anti-collision strip is further installed on the edge of the gantry 100 close to the treatment couch 200 to prevent the gantry 100 from touching the treatment couch 200 when swinging, which may affect the patient's treatment.

Through the aforementioned methods, patients can obtain a treatment space that is relatively fixed to the ground, and they will not feel the high-speed rotation of the gantry, thus providing a good patient experience. Non-coplanar irradiation is achieved through the swinging of the gantry, which adjusts the non-coplanar incidence angle during patient treatment. After positioning, the patient does not move, ensuring high accuracy in target volume localization and providing a good patient experience. The gantry can also perform irradiation while swinging, offering more incidence paths, allowing higher doses to be delivered to the tumor core volume, further improving the focal-to-skin ratio, and reducing the dose outside the target volume quickly, thus enabling better implementation of SBRT irradiation therapy.

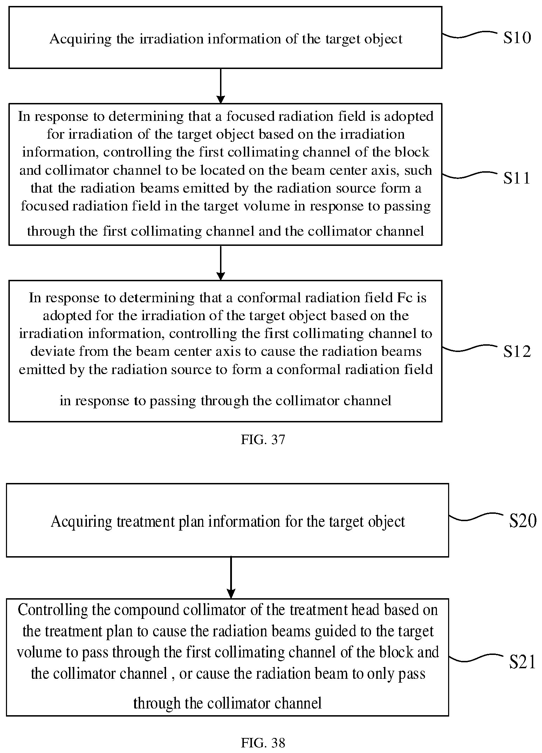

Furthermore, the embodiments of the present disclosure further provide a control method for a compound collimator, applicable for controlling the aforementioned compound collimator. Referring to FIG. 37, the method includes processes S10 to S12. Because the compound collimator is generally installed and operated in a radiotherapy system, the aforementioned method can also be referred to as a control method for a radiotherapy system.

In S10, the irradiation information of the target object is acquired.

In some embodiments, the irradiation information is the treatment plan information received by the upper computer or the lower computer. The acquired irradiation information of the target object includes the form of focused irradiation and conformal irradiation, so as to determine different irradiation forms based on different treatment plans.

In S11, in response to determining that a focused radiation field Ff is adopted for irradiation of the target object based on the irradiation information, the first collimating channel of the block and collimator channel are controlled to be located on the beam center axis, such that the radiation beams emitted by the radiation source form a focused radiation field in the target volume in response to passing through the first collimating channel and the collimator channel. In some embodiments, when using focused irradiation, it is further determined which size and specification of radiation field to adopt.

In some embodiments, in the case that the focused irradiation is employed, the movement of the block is controlled to make the first collimating channel of the block be located on the beam center axis; the movement of the leaves of the collimator is controlled to make the collimator channel of the collimator be located on the beam center axis; and finally, the radiation beams guided to the target volume pass through the first collimating channel of the block and the collimator channel, thus forming a focused radiation field Ff in the target volume.

In S12, in response to determining that a conformal radiation field Fc is adopted for the irradiation of the target object based on the irradiation information, the first collimating channel is controlled to deviate from the beam center axis to make the radiation beams emitted by the radiation source form a conformal radiation field Fc in response to passing through the collimator channel.

In some embodiments, in the case that a conformal radiation field Fc is adopted, the movement of the block is controlled to make the first collimating channel of the block deviate from the beam center axis; the movement of the leaves of the collimator is controlled to position the collimator channel of the collimator on the beam center axis, i.e., the beam center axis passes through the collimator channel; and then, the radiation beams pass through the collimator channel to form a conformal radiation field Fc in the target volume.

Through the aforementioned control method, based on different irradiation information, the collimator and block are controlled to generate the required focused radiation field Ff or conformal radiation field Fc. The entire process is simple and efficient.

By applying the aforementioned compound collimator to the radiotherapy system, the present embodiments of the present disclosure further provide a dose delivery method for the radiotherapy system, applicable for the dose delivery for the radiotherapy system. Referring to FIG. 38, the method includes processes S20 and S21:
In S20, treatment plan information for the target object is acquired.

In some embodiments, the treatment plan information is generated by the treatment planning system and sent to the upper computer; and the upper computer then distributes the treatment plan information to each lower computer and controls each component to execute the treatment plan information.

In some embodiments, the treatment plan information for the target object includes the use of coplanar irradiation or non-coplanar irradiation, as well as the use of focused irradiation or conformal irradiation, so as to determine the field formation method of the compound collimator and the swinging manner of the gantry 100 and treatment couch 200 based on different treatment plan information.

In S21, the compound collimator of the treatment head is controlled based on the treatment plan, such that the radiation beams guided to the target volume are caused to pass through the first collimating channel of the block and the collimator channel, or the radiation beam is caused to pass only through the collimator channel.

In the case that the radiation beams pass through the first collimating channel and the collimator channel, a precise focused radiation field Ff is formed; and in the case that the radiation beams only pass through the collimator channel, a conformal radiation field is formed. That is, based on the aforementioned treatment plan information, the field shaping method is selected.

In some embodiments, in some optional embodiments of the present disclosure, the dose delivery method for the radiotherapy system further includes: forming different angles between the treatment couch and the gantry by controlling the relative swing between the treatment couch and the gantry. Through the relative swing between the treatment couch and the gantry, coplanar or non-coplanar irradiation can be achieved to meet different treatment needs.

In some embodiments, the relative swinging between the treatment couch 200 and the gantry 100 is achieved by controlling the treatment couch 200 to swing along a horizontal plane, such that the radiation beams 10a emitted by the treatment head are focused on the target volume from different incident angles. In this way, through the swinging of the treatment couch 200, different angles are formed between the treatment couch 200 and the gantry 100, and the radiation beams 10a are emitted from different angles to achieve non-coplanar irradiation.

In some embodiments, the dose delivery method for the radiotherapy system further includes: controlling the gantry 100 to swing in the horizontal plane to make the radiation beams 10a emitted by the treatment head be focused on the target volume from different incident angles. At this time, through the swinging of the gantry 100, different angles are formed between the treatment couch 200 and the gantry 100, and the radiation beams 10a are emitted from different angles, achieving non-coplanar irradiation.

For the relative swinging method between the treatment couch 200 and the gantry 100, please refer to the aforementioned description for details.

Based on the patient's lesion, different radiation fields (conformal radiation field Fc or focused radiation field Ff) are selected. The two conformal modes can be quickly switched, and with one patient positioning, combined irradiation using the two modes can be adopted, providing a more flexible approach for clinical application. During creation of a treatment plan, one single radiation field is adopted or a combination of two radiation fields is adopted.

In the case that two types of fields are combined, one approach is to first use a conformal radiation field Fc based on a multi-leaf collimator (MLC) for basic dose irradiation, which covers a large volume; and then for the core lesion volume, a focused radiation field Ff combined with non-coplanar irradiation is configured for focused irradiation, increasing the dose within the target volume and improving the tumor cure rate. For patients suitable for SBRT, a precisely focused radiation field Ff combined with non-coplanar irradiation is employed. Of course, conventional conformal radiation field Fc intensity-modulated irradiation based on MLC can also be conducted using a compound collimator.

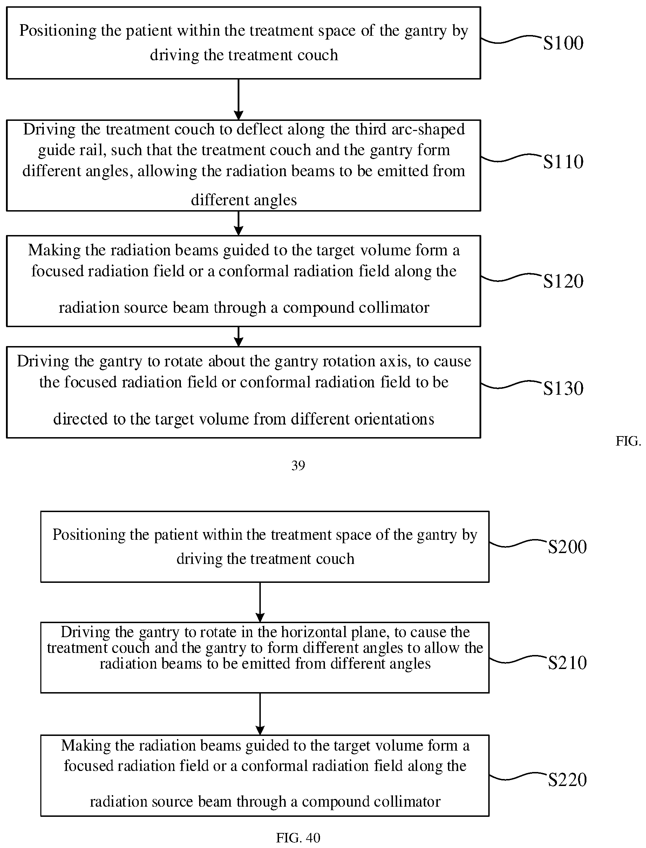

The embodiments of the present disclosure further provide a dose delivery method for a radiotherapy system. Referring to FIG. 39, the method includes processes S100 and S110:
In S100, the patient is positioned within the treatment space 100a of the gantry 100 by driving the treatment couch 200.

The treatment couch 200 is driven into the treatment space 100a by the first treatment couch driving device to prepare for treatment.

In S110, the treatment couch 200 is driven to deflect along the third arc-shaped guide rail 111a, such that the treatment couch 200 and the gantry 100 are caused to form different angles, allowing the radiation beams 10a to be emitted from different angles.

The treatment couch 200 is driven by the second treatment couch driving device to move along the third arc-shaped guide rail 111a, such that the treatment couch 200 is rotatable in the horizontal plane, allowing the treatment couch 200 to form different angles relative to the gantry 100.

Furthermore, the stability of the swinging motion of the treatment couch 200 is enhanced through the auxiliary action of the fourth arc-shaped guide rail 201 in some embodiments.

In some embodiments, referring to FIG. 39, the embodiments of the present disclosure provide a dose delivery method for a radiotherapy system, further including: in S120, making the radiation beams 10a guided to the target volume form a focused radiation field Ff or a conformal radiation field Fc along the radiation source beam through a compound collimator. In some embodiments, the compound collimator is the compound collimator provided in the present disclosure, which includes an aperture collimator (block 13) and a multi-leaf collimator (collimator 12); the compound collimator is driven to make the radiation beams 10a guided to the target volume pass through the aperture collimator to form a focused radiation field Ff; through the aforementioned block 13 and collimator 12 jointly forming a field, the radiation beams 10a pass through the collimator channel 12a and the first collimating channel 13a and form a focused radiation field Ff; or, the compound collimator is driven to make the radiation beams 10a guided to the target volume pass through the multi-leaf collimator to form a conformal radiation field Fc; or through the block 13 and collimator 12 jointly forming a field, the radiation beams 10a pass through the collimator channel 12a and form a conformal radiation field Fc.

The specific method for driving the compound collimator can be referred to the aforementioned embodiments of the present disclosure, and will not be elaborated here.

In some embodiments, referring to FIG. 39, the present disclosure provides a dose delivery method for a radiotherapy system. The dose delivery method further includes: in S130, by driving the gantry 100 to rotate about the gantry rotation axis 100b, making the focused radiation field Ff or conformal radiation field Fc be directed to the target volume from different orientations.

It should be noted that the order of processes in each flowchart is merely an example, and can be adjusted according to actual needs.

In practical applications, through the cooperation between the gantry 100 and the treatment couch 200, when moving the patient using the treatment couch 200, better irradiation can also be achieved by rotating the gantry 100.

In some embodiments, referring to FIG. 40, the present disclosure further provides a dose delivery method for a radiotherapy system. The dose delivery method includes processes S200 and S210.

In S200, the patient is positioned within the treatment space 100a of the gantry 100 by driving the treatment couch 200.

In S210, the gantry 100 is driven to rotate on the horizontal plane, such that the treatment couch 200 and the gantry 100 are caused to form different angles to allow the radiation beams 10a to be emitted from different angles.

In some embodiments, the second gantry driving device 120 drives the gantry 100 to rotate in the horizontal plane, such that the gantry 100 forms different angles relative to the treatment couch 200, achieving non-coplanar irradiation.

In some embodiments, the present disclosure further provides a dose delivery method for a radiotherapy system. The dose delivery method further includes: in S220, making the radiation beams 10a guided to the target volume form a focused radiation field Ff or a conformal radiation field Fc along the radiation source beam through a compound collimator.

In some embodiments, the compound collimator includes an aperture collimator (block) and a multi-leaf collimator (collimator), and the radiation beams 10a guided to the target volume forming a focused radiation field Ff or a conformal radiation field Fc along the radiation source beam through the compound collimator, includes:
driving the compound collimator to make the radiation beams 10a guided to the target volume pass through the aperture collimator (block) to form a focused radiation field Ff; or
driving the compound collimator to make the radiation beams 10a guided to the target volume pass through the multi-leaf collimator (collimator) to form a conformal radiation field Fc.

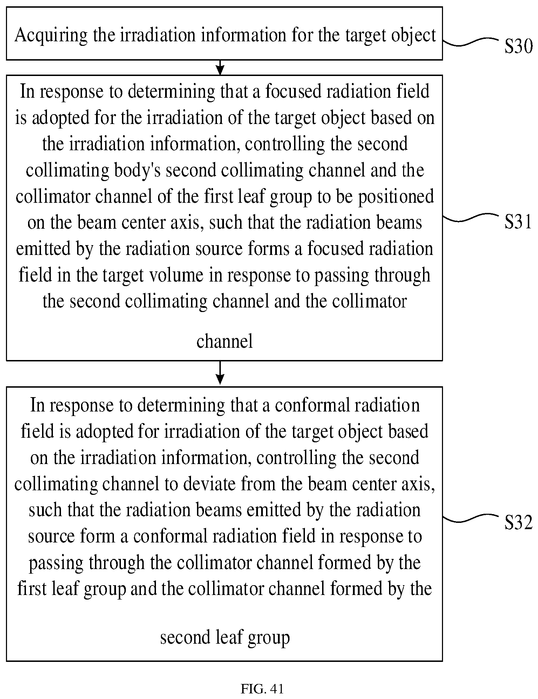

In other embodiments, for another compound collimator provided in the aforementioned embodiments, another control method for the compound collimator is also provided for controlling the aforementioned compound collimator, applicable for controlling the aforementioned compound collimator. Referring to FIG. 41, the method includes processes S30 to S32. Because the compound collimator is generally installed and operated in a radiotherapy system, the control method for the compound collimator here is actually a control method for the radiotherapy system.

In S30, the irradiation information for the target object is acquired. The specific details of process S30 are similar to those of process S10 in the aforementioned embodiment. For details, reference may be made to the above embodiments, which are not described herein any further.

In S31, in response to determining that a focused radiation field is adopted for the irradiation of the target object based on the irradiation information, the second collimating body's second collimating channel and the collimator channel of the first leaf group are controlled to be positioned on the beam center axis, such that the radiation beams emitted by the radiation source forms a focused radiation field in the target volume in response to passing through the second collimating channel and the collimator channel. In some embodiments, in the case that the focused irradiation is employed, which size and specification of radiation field to adopt may be further determined.

In some embodiments, in the case that the focused irradiation is employed, the movement of the leaves of the first leaf group is controlled to position the collimator channel of the first leaf group on the beam center axis; and the movement of the second collimating body is controlled to position the second collimating channel on the beam center axis. At this time, the second leaf group retreats away from the beam center axis to avoid the movement path of the second collimating body. The radiation beams pass through the collimator channel of the first leaf group and the second collimating channel, forming a focused radiation field in the target volume. After retracting from the beam center axis, the second leaf group approaches the beam center axis again, with the leaf ends of the second leaf group fitting the sides of the second collimating body to block leakage of surrounding radiation beams. The focused radiation field formed in this way is suitable for lesion volumes that are particularly small and have many surrounding organs at risk. That is, by controlling the compound collimator to allow the radiation beams guided to the target volume to pass through the second collimating channel and collimator channel, a focused radiation field is formed in the target volume.

In S32, in response to determining that a conformal radiation field is adopted for irradiation of the target object based on the irradiation information, the second collimating channel is controlled to deviate from the beam center axis, such that the radiation beams emitted by the radiation source form a conformal radiation field in response to passing through the collimator channel formed by the first leaf group and the collimator channel formed by the second leaf group.

In some embodiments, the radiation beams form a conformal radiation field in the target volume only by passing through a collimator channel formed by the first leaf group or a collimator channel formed by the second leaf group. In some embodiments, the radiation beams form a conformal radiation field in the target volume by passing through a collimator channel formed by the first leaf group and a collimator channel formed by the second leaf group, that is, the first leaf group and the second leaf group jointly form a conformal radiation field.

In some embodiments, in the case that a conformal radiation field is employed, the movement of the second collimating body is controlled to make the second collimating channel of the second collimating body deviate from the beam center axis; the movement of the leaves is controlled to make the collimator channels of the first leaf group and/or the second leaf group be positioned on the beam center axis; and the radiation beams pass through the collimator channels to form a conformal radiation field in the target volume.

Through the aforementioned control method, based on different irradiation information, the second collimating body and the first and second leaf groups are controlled to generate the required focused or conformal radiation fields. The entire process is simple and efficient.

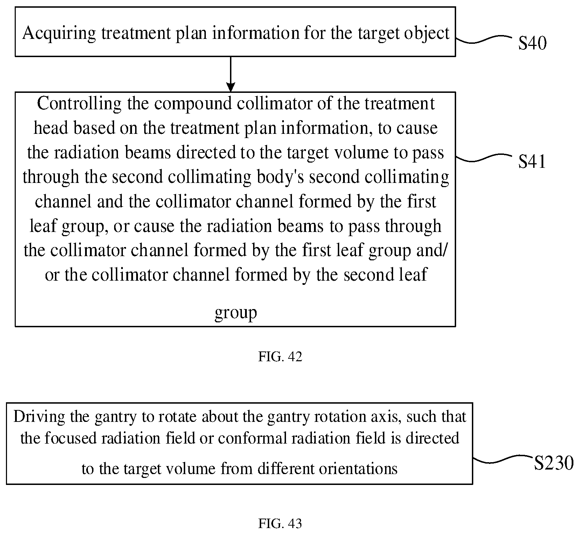

Applying the aforementioned compound collimator to the radiotherapy system, the embodiments of the present disclosure further provide a dose delivery method for the radiotherapy system, applicable for dose delivery of the radiotherapy system. Referring to FIG. 42, the method includes S40-S41.

In S40, treatment plan information for the target object is acquired.

The process S40 is similar to process S20 in the previous embodiment, and specific details can be referred to in the previous embodiment.

In S41, the compound collimator of the treatment head is controlled based on the treatment plan information, such that the radiation beams directed to the target volume are caused to pass through the second collimating body's second collimating channel and the collimator channel formed by the first leaf group, or pass through the collimator channel formed by the first leaf group and/or the collimator channel formed by the second leaf group.

In the case that the radiation beams pass through the second collimating channel and the collimator channel of the first leaf group, a precise focused radiation field is formed; and in the case that the radiation beams pass through the collimator channel formed by the first leaf group and/or the collimator channel formed by the second leaf group, a conformal radiation field is formed. That is, based on the aforementioned treatment plan information, the field shaping method is selected.

In this embodiment, different radiation fields (conformal radiation field or focused radiation field) can be selected based on the patient's lesion. The two radiation field modes can be quickly switched, and the patient is positioned once, allowing for combined irradiation using the two irradiation modes, providing a more flexible approach for clinical application. When creating a treatment plan, a single radiation field is adopted, or a combination of two radiation fields is adopted.

In the case that two types of radiation fields are used in combination, a conformal radiation field is first formed based on the first leaf group and/or the second leaf group for basic dose irradiation, covering a large volume; and for the core volume of the lesion, a focused radiation field combined with non-coplanar irradiation is used for focused irradiation, increasing the dose within the target volume and improving the tumor cure rate. For patients suitable for SBRT, a precise focused radiation field combined with non-coplanar irradiation is employed. Of course, conventional conformal intensity-modulated radiation therapy can also be conducted using a compound collimator.

In this embodiment, the compound collimator includes a first leaf group and a second leaf group arranged sequentially along the beam center axis direction. The first and second leaf groups each include a plurality of leaves, and the movement of the plurality of leaves form a collimator channel allowing the radiation beams to pass through. A second collimating body is further disposed in the same layer as the second leaf group and orthogonally arranged relative to the second leaf group. The second collimating body is provided with a second collimating channel. The second collimating body is moveable relative to the beam center axis. In the case that the second collimating body moves to a position where the second collimating channel is aligned with the beam center axis, the radiation beams pass through the collimator channel formed by the first leaf group and the second collimating channel to form a focused radiation field in the target volume, which is used for the treatment of particularly small lesion volumes with many surrounding organs at risk. The second leaf group approaches the beam center axis, and the leaf ends of the second leaf group fit closely to both sides of the second collimating body, thereby blocking leakage of the surrounding radiation beams. In the case that the second collimating body moves such that the second collimating channel deviates from the beam center axis, the radiation beams pass through the collimator channel formed by the first leaf group and/or the collimator channel formed by the second leaf group to form a conformal radiation field in the target volume, which is used for the treatment of complex large-radiation-field lesions. At this time, in the case that only one leaf group is located at the beam center axis and the radiation beam passes through its collimator channel, the other leaf group that has not moved to the beam center axis can serve to block leakage of radiation outside the radiation field. In other words, the compound collimator of this embodiment enables switching between different radiation fields, improves adaptability, significantly enhances treatment efficiency, and provides a guarantee for improving the cure rate.

In some embodiments, referring to FIG. 43, the present disclosure further provides a dose delivery method for a radiotherapy system. The method further includes S230: driving the gantry 100 to rotate about the gantry rotation axis 100b, such that the focused radiation field Ff or conformal radiation field Fc is directed to the target volume from different orientations. The relevant process of this control method is similar to those described above and can be implemented with reference.

In some embodiments, in addition to the aforementioned methods of swinging the treatment couch 200 or the gantry 100, different angles are formed between the treatment couch 200 and the gantry 100 by simultaneously swinging both of them. Specific details refer to the aforementioned methods of swinging the treatment couch 200 and the gantry 100, which will not be elaborated here.

In some embodiments, different irradiation methods are adopted during irradiation: the gantry 100 and the treatment couch 200 move relative to each other to form an angle, and the gantry 100 continuously rotates around the gantry rotation axis 100b to perform non-coplanar irradiation. The radiotherapy system of this embodiment supports dynamic stereotactic irradiation. During the irradiation process, the relative angular position of the gantry 100 and the treatment couch 200 can be adjusted in real time to achieve more incident angles, improve the focal-to-skin ratio, and enhance the dose gradient around the target volume.

In some embodiments, the two radiation field modes can be flexibly switched. One option is to use the conformal radiation field Fc for efficient basic dose irradiation, and then employ the precise focused radiation field Ff to irradiate the core volume within the target volume, which better meets the dose distribution requirements of SBRT.

Traditional fixed-field irradiation can only achieve conformal irradiation and cannot adjust intensity. The compound collimator provided in the present disclosure can adjust intensity within the fixed radiation field, achieving precise shape and dose distribution.

In some embodiments, after the gantry 100 and the treatment couch 200 reaching a relative position by adjustments, an arc irradiation is further performed. During the irradiation process, based on the planned dose distribution requirements, a single field mode is selected, or a combination of two field modes is chosen.

The embodiments provided in the present disclosure achieve stereotactic irradiation and rotational intensity-modulated therapy by coordinating the movement of the aforementioned gantry 100 and treatment couch 200, thereby obtaining more incident angles. The incident angles are adjusted based on the location, shape, and surrounding organ distribution of the lesion, maximizing the protection of surrounding normal organs and delivering the dose to the target volume. Based on the combination of two radiation field modes, each mode's advantages are leveraged to enhance treatment precision, thereby improving the overall treatment effect of the radiotherapy system.

The above description is merely some embodiments of the present disclosure and is not intended to limit the scope of protection of the present disclosure. For those skilled in the art, various modifications and variations can be made to the present disclosure. Any modifications, equivalent substitutions, improvements, etc., within the concept and principles of the present disclosure shall be included within the scope of protection of the present disclosure.

## Claims

1. A radiotherapy system, comprising: a radiation source, a treatment couch, and a gantry, wherein the radiation source is configured to emit radiation beams and is mounted on the gantry; and the treatment couch is configured to carry a patient and move the patient to a predetermined position;
wherein the radiotherapy system further comprises a first gantry driving device and a second gantry driving device, wherein the first gantry driving device is configured to drive the gantry to rotate about a gantry rotation axis, and the second gantry driving device is configured to drive the gantry to rotate about a first axis as a rotation axis, the first axis being perpendicular to a horizontal plane;
wherein the second gantry driving device is higher than a lowest point of the rotation of the gantry.

2. The radiotherapy system according to claim 1, further comprising a base, wherein the gantry is disposed on the base and is rotatably connected to the base via the second gantry driving device, such that the gantry is rotatable relative to the base.

3. The radiotherapy system according to claim 2, wherein the second gantry driving device comprises an arc-shaped guide rail, a slide block, and a drive; wherein the arc-shaped guide rail is arranged on the base, the slide block is arranged on the gantry, and the drive is configured to drive the slide block to move along the arc-shaped guide rail; and the first axis is a rotation axis of the arc-shaped guide rail, and the first axis passes through an isocenter of the radiotherapy system.

4. The radiotherapy system according to claim 3, wherein the arc-shaped guide rail comprises a first arc-shaped guide rail and a second arc-shaped guide rail arranged oppositely, the first arc-shaped guide rail and the second arc-shaped guide rail being coaxial; and
the slide block comprises a first guide rail slide block and a second guide rail slide block, wherein the first guide rail slide block and the second guide rail slide block are separately connected to the gantry; or the first guide rail slide block and the second guide rail slide block are connected by a connecting piece, the connecting piece being connected to the gantry.

5. The radiotherapy system according to claim 4, wherein the first guide rail slide block is driven by the drive to move along the first arc-shaped guide rail and drive the second guide rail slide block to move along the second arc-shaped guide rail.

6. The radiotherapy system according to claim 3, wherein the drive is mounted on the gantry, and the drive is a gear drive, wherein the gear drive comprises a motor, a gear, and a gear ring, the gear ring being coaxial with the arc-shaped guide rail, and the motor driving the gear to move along the gear ring.

7. The radiotherapy system according to claim 1, further comprising: an imaging system arranged on the gantry.

8. The radiotherapy system according to claim 3, wherein the arc-shaped guide rail or the slide block is higher than the lowest point of the rotation of the gantry.

9. The radiotherapy system according to claim 4, wherein the first arc-shaped guide rail and the second arc-shaped guide rail are respectively disposed on two sides of the gantry.

10. The radiotherapy system according to any one of claims 1 to 9, wherein the treatment couch is rotatable about the first axis as the rotation axis.

11. The radiotherapy system according to claim 10, wherein
a treatment space is formed in the gantry, and the gantry drives the radiation source to rotate about the gantry rotation axis; and
a bottom of the treatment couch is configured to be connected to a third arc-shaped guide rail; and the treatment couch comprises a first treatment couch driving device and a second treatment couch driving device, wherein the first treatment couch driving device is configured to drive the treatment couch into the treatment space, and the second treatment couch driving device is configured to drive the treatment couch to move along the third arc-shaped guide rail, such that the treatment couch is rotatable about the first axis as the rotation axis.

12. The radiotherapy system according to claim 11, wherein the first axis passes through a center of the third arc-shaped guide rail and an isocenter of the radiotherapy system.

13. The radiotherapy system according to claim 11, further comprising: a base, wherein the gantry is arranged on the base, and the third arc-shaped guide rail is arranged on the base.

14. The radiotherapy system according to claim 11, wherein a fourth arc-shaped guide rail is further arranged at the bottom of the treatment couch to assist in supporting the rotation of the treatment couch; and the treatment couch is connected to ground via the fourth arc-shaped guide rail.

15. The radiotherapy system according to claim 14, wherein the third arc-shaped guide rail and the fourth arc-shaped guide rail are concentrically arranged.

16. The radiotherapy system according to claim 14, wherein the treatment couch is connected to the third arc-shaped guide rail and the fourth arc-shaped guide rail respectively via slide blocks.

17. The radiotherapy system according to any one of claims 1 to 16, further comprising: a compound collimator, wherein the compound collimator is configured to shape the radiation beams emitted by the radiation source to form a focused radiation field or a conformal radiation field.

18. The radiotherapy system according to claim 17, wherein the compound collimator comprises a collimator and a block that are arranged sequentially along a direction of a beam center axis; wherein
the collimator comprises a plurality of leaves, wherein the plurality of leaves forms, by movements, a collimator channel allowing the radiation beams to pass through; and
a first collimating channel is formed in the block, and the block is moveable relative to the beam central axis; wherein in a case that the block moves to enable the beam central axis to pass through the first collimating channel, the radiation beams form the focused radiation field in a target volume in response to passing through the collimator channel and the first collimating channel, and in a case that the block moves to an evasive position, the radiation beams form the conformal radiation field in the target volume in response to passing through the collimator channel.

19. The radiotherapy system according to claim 18, wherein the block comprises a block body and a first collimating body that is disposed on the block body and movable relative to the block body, wherein the block body is configured to drive the first collimating body to translate, the first collimating channel is disposed in the first collimating body, and a block channel for coaxial correspondence with the first collimating channel is arranged in the block body;
wherein by translation of the block body and movement of the first collimating body, the first collimating channel and the block channel are made to deviate from the beam center axis, or the beam center axis is made to pass through the first collimating channel and the block channel.

20. The radiotherapy system according to claim 19, wherein the first collimating body is cylindrical, and a plurality of the first collimating channels are arranged in the first collimating body, wherein the plurality of the first collimating channels have different aperture sizes.

21. The radiotherapy system according to claim 20, wherein one block channel is arranged in the block body, and the block channel corresponds to the plurality of first collimating channels; or
a plurality of block channels are arranged in the block body, and the plurality of block channels are in one-to-one correspondence with the plurality of first collimating channels.

22. The radiotherapy system according to claim 19, wherein the first collimating body rotates such that the first collimating channel coaxially corresponds to the block channel or the first collimating channel is deviated from the block channel.

23. The radiotherapy system according to claim 19, wherein a translation direction of the block body is parallel to a movement direction of the leaves of the collimator, or the translation direction of the block body is perpendicular to the movement direction of the leaves of the collimator; or a movement direction of the first collimating body is parallel to the movement direction of the leaves of the collimator, or the movement direction of the first collimating body is perpendicular to the movement direction of the leaves of the collimator.

24. The radiotherapy system according to claim 19, wherein in a case that the first collimating channel deviates from the beam center axis, an angle between an axis of the first collimating channel and the beam center axis is 90 degrees.

25. The radiotherapy system according to claim 19, wherein the block comprises two block components that are oppositely arranged, wherein the first collimating channel is arranged in at least one of the two block components, or arranged in each of the two block components.

26. The radiotherapy system according to claim 25, wherein in a case that the first collimating channel is arranged in each of the two block components, the first collimating channel of one of the two block components is larger than the first collimating channel of the another of the two block components.

27. The radiotherapy system according to claim 18, wherein the block comprises a first block group and a second block group that are orthogonally arranged and translatable, the first collimating channel being formed in the first block group; wherein in a case that the first collimating channel is deviated from the beam center axis or the beam center axis pass through the first collimating channel by translation of the first block group, the second block group is configured to shield a gap formed at ends of leaves of the collimator.

28. The radiotherapy system according to claim 27, wherein a translation direction of the first block group is parallel to a movement direction of the leaves of the collimator.

29. The radiotherapy system according to claim 27, wherein the first block group and the second block group are located on a same plane, or the first block group and the second block group are located on different planes.

30. The radiotherapy system according to claim 27, wherein a plurality of first collimating channels are arranged, wherein the plurality of first collimating channels have different aperture sizes, and different first collimating channels correspond to the beam center axis by translation of the first block group.

31. The radiotherapy system according to any one of claims 28 to 30, wherein the first collimating channel at least comprises a cylindrical channel or a conical channel, wherein a cross-sectional size of an end of the conical channel close to the radiation source is smaller than a cross-sectional size of an end of the conical channel away from the radiation source.

32. The radiotherapy system according to claim 17, wherein the compound collimator comprises a first leaf group and a second leaf group that are arranged sequentially along a direction of a beam center axis, and a second collimating body arranged orthogonally to the second leaf group on the same layer as the second leaf group; wherein
the first leaf group and the second leaf group each comprise a plurality of leaves, and movements of the plurality of leaves form collimator channels allowing the radiation beams to pass through; and
a second collimating channel is arranged in the second collimating body, and the second collimating body is moveable relative to the beam central axis; wherein in a case that the second collimating body moves to enable the beam central axis to pass through the second collimating channel, the radiation beams form a focused radiation field in a target volume in response to passing through a collimator channel formed by the first leaf group and the second collimating channel; and in a case that the second collimating body moves to an evasive position, the radiation beams form a conformal radiation field in the target volume in response to passing through the collimator channel formed by the first leaf group and/or a collimator channel formed by the second leaf group.

33. The radiotherapy system according to claim 32, wherein the first leaf group is arranged close to the radiation source, and the second leaf group is arranged on a side away from the radiation source.

34. The radiotherapy system according to claim 33, wherein in the case that the second collimating body moves to allow the beam central axis to pass through the second collimating channel, ends of the leaves of the second leaf group fit both sides of the second collimating body, so as to block leakage of the radiation beams.

35. The radiotherapy system according to claim 34, wherein a side wall of the second collimating body fitting the ends of the leaves is a concave side wall.

36. The radiotherapy system according to claim 33, wherein the leaves of the first leaf group and the leaves of the second leaf group are arranged to overlap with each other, and the leaves of the second leaf group block gaps between the leaves of the first leaf group.

37. The radiotherapy system according to claim 32, wherein the first leaf group and the second leaf group are respectively arranged in two boxes, or the first leaf group and the second leaf group are arranged in a same box.

38. The radiotherapy system according to claim 32, wherein the second collimating channel comprises at least a cylindrical channel or a conical channel, wherein a cross-sectional size of an end of the conical channel close to the radiation source is smaller than a cross-sectional size of an end of the conical channel away from the radiation source.

39. The radiotherapy system according to any one of claims 32 to 38, wherein the second collimating body is provided with a plurality of second collimating channels, wherein the plurality of second collimating channels have different aperture sizes.

40. The radiotherapy system according to claim 39, wherein the second collimating body comprises two collimating sub-bodies that are oppositely arranged, wherein the second collimating channel is arranged in at least one of the two collimating sub-bodies, or arranged in each of the two collimating sub-bodies.

41. The radiotherapy system according to claim 40, wherein in a case that each of the two collimating sub-bodies is provided with the second collimating channel, a second collimating channel of one of the two collimating sub-bodies is larger than a second collimating channel of the another of the two collimating sub-bodies.

42. The radiotherapy system according to claims 32 to 41, further comprising: a guide rail, wherein the second collimating body is arranged on the guide rail.

43. The radiotherapy system according to claims 32 to 41, further comprising a rotational disc, wherein the compound collimator is arranged on the rotational disc and is driven to rotate by the rotational disc.

44. A dose delivery method for a radiotherapy system, applicable for dose delivery for the radiotherapy system according to any one of claims 1 to 43, the method comprising:
Positioning a patient within a treatment space of the gantry by driving the treatment couch;
driving the gantry to rotate about the first axis as the rotation axis to cause the treatment couch and the gantry to form different angles, so as to allow radiation beams to be emitted from different angles, the first axis being perpendicular to a horizontal plane; and
driving the gantry to rotate about the gantry rotation axis to cause the radiation beams to be directed to a target volume from different orientations.

45. The method according to claim 44, further comprising:
driving the treatment couch to rotate about the first axis as the rotation axis to cause the treatment couch to form different angles relative to the gantry.

46. The method according to claim 44, further comprising:
controlling, by a compound collimator, the radiation beams guided to the target volume to form a focused radiation field or a conformal radiation field.

47. A dose delivery method for a radiotherapy system, applicable for dose delivery for the radiotherapy system according to any one of claims 18-31, the method comprising:
acquiring treatment plan information for a target subject; and
controlling the compound collimator of a treatment head based on the treatment plan to cause radiation beams guided to a target volume to pass through the first collimating channel of the block and the collimator channel, or cause the radiation beams to only pass through the collimator channel.

48. A dose delivery method for a radiotherapy system, applicable for dose delivery for the radiotherapy system according to any one of claims 32 to 43, the method comprising:
acquiring treatment plan information for a target subject; and
controlling the compound collimator of a treatment head based on the treatment plan information to cause radiation beams guided to a target volume to pass through the second collimating channel of the second collimating body and the collimator channel formed by the first leaf group, or cause the radiation beams to pass through the collimator channel formed by the first leaf group and/or the collimator channel formed by the second leaf group.

49. A control method for a radiotherapy system, applicable for controlling the radiotherapy system according to any one of claims 18 to 31, the method comprising:
acquiring irradiation information for a target object;
in response to determining that a focused radiation field is adopted for irradiation of the target object based on the irradiation information, controlling the block to move to enable the beam center axis to pass through the first collimating channel and collimator channel, such that the radiation beams emitted by the radiation source form the focused radiation field in a target volume in response to passing through the first collimating channel and collimator channel; and
in response to determining that a conformal radiation field is adopted for irradiation of the target object based on the irradiation information, controlling the block to move to an evasive position, such that the radiation beams emitted by the radiation source form the conformal radiation field in response to passing through the collimator channel.

50. A control method for a radiotherapy system, applicable for controlling the radiotherapy system according to any one of claims 32 to 43, the method comprising:
acquiring irradiation information for a target object;
in response to determining, based on the irradiation information, that a focused radiation field is adopted for irradiation of the target object, controlling the second collimating body and the first leaf group to move to allow the beam center axis to pass through the second collimating channel of the second collimating body and the collimator channel of the first leaf group, such that the radiation beams emitted by the radiation source form the focused radiation field in a target volume in response to passing through the second collimating channel and the collimator channel; and
in response to determining, based on the irradiation information, that a conformal radiation field is adopted for irradiation of the target object, controlling the second collimating body to move to an evasive position, such that the radiation beams emitted by the radiation source form the conformal radiation field in response to passing through the collimator channel formed by the first leaf group and the collimator channel formed by the second leaf group.
